(19) Europäisches Patentamt
European Patent Office
Office européen des brevets

(11) EP 1 862 927 B1

(12) EUROPEAN PATENT SPECIFICATION

(45) Date of publication and mention of the grant of the patent:
**07.10.2009 Bulletin 2009/41**

(51) Int Cl.:
***G06F 19/00*** *(2006.01)*

(21) Application number: **06011342.0**

(22) Date of filing: **01.06.2006**

(54) **Descriptors of three-dimensional objects, uses thereof and a method to generate the same**

Deskriptoren für dreidimensionale Objekte, deren Verwendung und Verfahren zu deren Erstellung

Descripteurs d'objets tridimensionnels, ses utilisations et procédé pour les générer

(84) Designated Contracting States:
**AT BE BG CH CY CZ DE DK EE ES FI FR GB GR HU IE IS IT LI LT LU LV MC NL PL PT RO SE SI SK TR**
Designated Extension States:
**AL BA HR MK YU**

(43) Date of publication of application:
**05.12.2007 Bulletin 2007/49**

(73) Proprietor: **SILICOS NV**
**3590 Diepenbeek (BE)**

(72) Inventors:
- **De Winter, Hans Louis Jos**
  **2970 Schilde (BE)**
- **Langenaeker, Wilfried Gert Roger**
  **3720 Kortessem (BE)**

(74) Representative: **Bird, William Edward et al**
**Bird Goën & Co.**
**Klein Dalenstraat 42A**
**3020 Winksele (BE)**

(56) References cited:
- **LANGENAEKER ET AL.: "Spectrophore technology for fast and reliable virtual screening" ABSTRACTS OF PAPERS AMERICAN CHEMICAL SOCIETY, 26 March 2006 (2006-03-26), page 361, XP007900845**
- **KUPPENS T ET AL: "Determination of the stereochemistry of 3-hydroxymethyl-2,3-dihydro-[ 1,4]dioxino[2,3-b]pyridine by vibrational circular dichroism and the effect of DFT integration grids" JOURNAL OF PHYSICAL CHEMISTRY A ACS USA, vol. 107, no. 4, 30 January 2003 (2003-01-30), pages 542-553, XP002432609 ISSN: 1089-5639**
- **SANTOS-FILHO OSVALDO A ET AL: "The 4D-QSAR paradigm: Application to a novel set of nonpeptidic HIV protease inhibitors" QUANTITATIVE STRUCTURE-ACTIVITY RELATIONSHIPS, vol. 21, no. 4, October 2002 (2002-10), pages 369-381, XP002432610 ISSN: 0931-8771**
- **EISENSTEIN MIRIAM ET AL: "On proteins, grids, correlations, and docking" COMPTES RENDUS BIOLOGIES, vol. 327, no. 5, May 2004 (2004-05), pages 409-420, XP004512381 ISSN: 1631-0691**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 1 862 927 B1

## Description

Field of invention

The invention relates to the field of molecular modelling for drug discovery and to the application of virtual screening algorithms for chemical discovery and in particular to the use of computer based systems. In particular, a method is disclosed to generate descriptors, e.g. to transform any type of three-dimensional objects with associated properties, such as molecular species with their associated atomic properties, into strings of real numbers. Once obtained, these descriptors can be used to understand and derive structure/activity relationships, virtual screening of molecular databases, and virtual synthesis of molecules with predefined properties as well as lead to synthesis of such compounds. In addition to its application on small molecules, the method can also be applied to the binding pockets of proteins or other biomolecules, e.g. by converting this pocket into its mirror image.

Background of the invention

Traditionally, the design of novel molecular species (e.g. drugs) has essentially been a trial-and-error process despite the tremendous efforts devoted to it by pharmaceutical and academic research groups. In an attempt to counter the rapidly increasing costs associated with the discovery of new medicines, new computer-based approaches are conducted. Modern approaches to computer-aided molecular design fall into two general categories. The first includes structure-based methods which utilise the three-dimensional structure of a ligand-bound receptor. The second approach includes ligand-based methods in which the physicochemical or structural properties of ligand molecular species are characterized. A classic example of this concept is a quantitative structure-activity relationship (QSAR) model. Quantitative structure-activity relationships are mathematical relationships linking chemical structures - represented in the form of molecular descriptors - and pharmacological activity in a quantitative manner for a series of molecular species.

Virtual screening is the computational process whereby libraries of existing or virtual molecular species are searched for molecular species that meet well-defined criteria. In general, virtual screening is applied to search for molecular species that might be active against certain disease related proteins, whereas the activity is derived from the calculated interaction between the protein and the molecular species. Scoring of the molecular species is performed using well-defined mathematical functions with the aim to prioritize these molecular species for further analysis. Typically, two major virtual screening tendencies can be distinguished.

The first tendency consists of the protein structure-based approach whereby the potential binding pocket of a protein is used as reference function. The selection of potential binding pockets is still a major challenge within the pharmaceutical industry. Once the reference function is known, one can start with the screening of molecular species having the desired properties with respect to binding to the target protein. A number of suitable methods have been described:

- Docking of molecular species within the target protein.
- Pharmacophore representation of the binding pocket of the target protein.

The second tendency consists of a ligand-based approach whereby molecular species with known affinity for a target protein or disease model are used as reference function. A model is derived from these reference molecular species and can be used to annotate other molecular species with respect to their potential binding capabilities. A number of suitable ligand-based approaches have been described. Some are two-dimensional and some are three-dimensional. The 2D methods have the advantage of being applied very efficiently to search molecular databases. The disadvantage is that they are rather unspecific, which is not the case for the somewhat slower 3D methods. These forms of virtual screening can be integrated with available high-throughput screening (HTS) results. Below is provided an overview of a typical ligand-based virtual screening application which is combined with high-throughput screening:-

1. Select a training set of molecular species from the HTS results;
2. Train a model based on common characteristics of the selected molecular species;
3. Use the model to score the other molecular species within the database;
4. Validate the prioritized molecular species using the HTS results or by means of new biochemical assay data;
5. Repeat the procedure until convergence of the model has been reached.

Such modern high-throughput screening platforms requires the implementation and integration of efficient and robust virtual screening protocols and algorithms.

In order to be suitable for use within a computational context, molecular information must be translated into a suitable form, generally called a descriptor. Molecular descriptors can vary greatly in their complexity. A simple example

may be a structural key descriptor, which takes the form of a binary indicator variable that encodes the presence of certain substructure or functional features. Other descriptors, such as HOMO (Highest Occupied Molecular Orbital) and LUMO (Lowest Unoccupied Molecular Orbital) energies, require semi-empirical or quantum mechanical calculations and are therefore more time-consuming to compute. Molecular descriptors are often categorised according to their dimensionality, which refers to the structural representation from which the descriptor values are derived. In general, one can classify the current molecular descriptors as one dimensional (1 D), two dimensional (2D), or three dimensional (3D).

One dimensional descriptors are a reflection of the 'bulk' properties of molecular species, like the molecular weight, the number of atoms, or the molecular distribution between hydrophilic and lipophilic phases. One dimensional descriptors are generally fast to calculate and can be calculated from the molecular composition alone. Nevertheless, one dimensional descriptors lack any information about the molecular connectivity between the atoms, and are therefore rather of limited accuracy when applied to drug discovery and virtual screening problems.

The calculation of two dimensional descriptors requires knowledge of the molecular topology, and comprises information on the presence or absence of well-defined functional moieties, topological distances between well-defined atoms, and information regarding sidechains and ringsystems. Two dimensional descriptors have found their use in chemical similarity analyses and structure-activity relationships, and are useful in complementing three dimensional descriptors. The most widely used two-dimensional descriptors are molecular fingerprints, 'E-state' indices, and hologram QSAR descriptors.

Molecular fingerprints are essentially bitmaps consisting of on- and offbits, where each position along the bitmap is assigned to a specific and well-defined molecular fragment. If that particular fragment exists in the molecular species under consideration, then the corresponding bit is set to on, otherwise it is left as off. There are two general methods of 2D fingerprint generation. The first, known as the 'hashed' method, uses a set of rules for generating the fragments for fingerprinting. The second method, known as the 'keyed' method, requires a priori substructural definitions for all fragments that should be searched for during the fingerprint generation process. Similarity assessments between molecular species based on two dimensional fingerprints can be done in a number of ways, although the most commonly used metrics are based on Tanimoto coefficients. The Tanimoto coefficient compares the number of fingerprint bits in common between pairs of structures.

Electrotopological state (E-state) indices capture both molecular connectivity and the electronic character of a molecular species. The method makes use of the hydrogen-suppressed graph to represent the molecular structure. The focus of the method is on the individual atoms and hydride groups of the molecular skeleton. Intrinsic valence and sigma electron descriptors are assigned to each atom depending on the counts of valence and sigma electrons of the corresponding atoms. From these atom descriptors molecular connectivity indices may be calculated by multiplying the sigma and valence values for each atom in a fragment within a molecular species. This product is then converted to the reciprocal square root and called the connectivity subgraph term.

Hologram QSAR (HQSAR) is another two dimensional descriptor approach in which counts are made of the number of times each fragment is encountered in a molecular species, rather than merely using bitmaps to represent the absence or presence of particular fragments. The resulting integer strings are subsequently hashed to reduce string length and used as input for Partial Least Squares analysis to correlate with biological data.

Three-dimensional descriptors are a reflection of the molecular shape and of the spacial arrangements of the functional moieties which are thought to be important for the interaction between ligand and receptor. As implied by the name, three-dimensional descriptors are generated from a three dimensional representation of molecular species. With very few exceptions, the descriptor values are computed from a static conformation, which is either a standard conformation with ideal geometries generated from programs such as CORINA (Sadowski et al., 1993, Chem. Rev. 7, 2567-2581) or Omega (Boström et al., 2003, J. Mol. Graph. Mod. 21, 449-462), or a conformation that is fitted against a target X-ray structure or a pharmacophore.

An example of three-dimensional descriptor is described in US5025388, which relates to the CoMFA methodology. The CoMFA methodology, which is an acronym for Comparative Molecular Field Analysis, is a 3D quantitative structure-activity relationship technique which ultimately allows one to design and predict activities of molecular species. The database of molecular species with known properties, the training set, are suitably aligned in 3D space according to various methodologies. Charges are then calculated for each molecular species at a level of theory deemed appropriate. Steric and electrostatic fields are subsequently calculated for each molecular species by interaction with a probe atom at a series of grid points surrounding the aligned database in three-dimensional space. Finally, correlation of these field energy terms with a property of interest is performed by means of partial least squares with cross-validation, giving a measure of the predictive power of the model.

The CoMFA method has the inconvenience that it requires the alignment of the molecules of investigation in the same reference frame, which makes the applicability of CoMFA to molecular systems of different structural classes difficult. It also has the inconvenience not to permit the discrimination between stereoisomers, additionally, the descriptors obtained by this method only translate the electronic properties of the molecular species. There is therefore a need in

the art for an improved, stereospecific and fast method of generating descriptors from three-dimensional objects by translating a wider range of their properties. There is also a need in the art for such a method not requiring alignment of the molecules under investigation.
Wilfried Langenaeker, spectrophore™ technology for fast and reliable virtual screening, 231st ACS National Meeting, Atlanta, GA, March 26-30, 2006, announces a new technology which allows to rapidly convert three-dimensional molecular properties into one-dimensional molecular fingerprints. It announces
that typical molecular properties that can be converted into Specrtroghores™ include electrostatic potentials atomic lipophilocities, and hard- and softness potentials. It announces that molecules with similar 3D-properties, and as such similar biological activites, will always yield similar Spectrophores™. It announces that therefore this technology is well suited as a rapid and accurate virtual screening tool.
Kuppens T. et al. "Determination of the stereochemistry of 3-hydroxymethyl-2,3-dihydro-[1,4]dioxino[2,3]-blpyridine by vibrational circular dichroism and the effect of DFT integration grids" JOURNAL OF PHYSICAL CHEMISTRY A ACS USA vol. 107, no. 4. (2003-01-30), pages 542-553 discloses a method for determining the stereochemistry of 3-hydroxymethyl-2,3-dihydro-[1,4]dioxino[2,3-b]pyridine. The method comprises calculating the theoretical vibrational circular dichroism spectra of 3-hydroxymethyl-2,3-dihydro-[1,4]dioxino[2,3-b]pyridine via the use of integration grids.

## Summary

The present invention has the object to provide methods for molecular modelling, e.g. for drug discovery or for virtual screening, for molecular discovery and in particular to the use of computer based systems. In particular, the present invention provides methods for generating descriptors, e.g. to transform any type of three-dimensional objects with associated properties, such as molecular species with their associated atomic properties, into strings of real numbers. A further object of the present invention is to use the descriptors to understand and derive structure/activity relationships, virtual screening of molecular databases, and virtual synthesis of molecules with predefined properties as well as to lead to synthesis of such compounds. A further object is the provision of a method for investigating the binding to pockets of proteins or other biomolecules such as molecules or substances that have an antigenic determinant.

The present invention provides a method as defined in claim 1 to transform three dimensional objects, said three dimensional objects being selected from the group consisting of

1) a surface obtained from a biomolecule pocket,
2) a three dimensional configuration of a molecular species, and
3) a catalytic surface,

generally represented by a set of atomic coordinates in three-dimensional space, and the associated properties of these atoms as represented by real or integer numbers, into one dimensional strings of real numbers. The method makes use of a computer or a computing system. In the case of molecular species, the method is based on the evaluation, in an automated fashion, of the interaction of molecular species with an artificial environment implemented as a set of cages surrounding the molecular species. The invention results from the unexpected finding that pairs of molecular species that interact in a similar way with a set of these cages, are also interacting in a similar way with their natural environment, which could be expressed as, for example, the binding affinity to protein receptors, or the penetration through the blood-brain barrier.

When applied to molecules, a process in accordance with the present invention involves a number of steps. The first step is the generation of the three dimensional coordinates of the molecule and the calculation or assignment of properties to each of the atoms. Secondly, a set of cages is generated which are then used, in the third and final phase, to evaluate the interaction of the molecules with each of the cages. Each of the obtained interaction values is stored as a separate descriptor interaction value $V_i$. Pairs of descriptors can then be compared by means of a suitable distance measure, for instance, simple Euclidean distance calculations.

When applied to molecules, the present invention should speed up the process of drug design, discovery, and identification by allowing researchers to characterise molecules with respect to their potential of interacting with their environment, being for example protein active sites, aquatic environment, or biological membranes. Applications of the invention can be found in virtual screening and QSAR model building, ligand- and protein-based drug design, and molecular database clustering and characterisation.

The present invention includes computer program products such as software for implementing any of the methods of the invention and a machine-readable data or signal carrier storing an executable program which implements any of the methods of the present invention when executed on a computing device. Such a data carrier may be a magnetic storage device such as a diskette, hard driven magnetic tape or an optical data carrier such as a DVD or CD-ROM, solid state memory such as a USB memory stick, flash memory, etc.

Brief description of the figures

Figure 1 is a flowchart showing a process of the generation of descriptors according to an embodiment of the present invention.

Figure 2 is a flowchart showing a process of the generation of descriptors when applied to molecular species according to an embodiment of the present invention.

Figure 3 is a flowchart showing the generation of a single descriptor interaction value $V_i$ (i.e. a descriptor point) when applied to molecular species according to an embodiment of the present invention.

Figure 4 shows four examples of cages with their corresponding spatial distribution of property values $V_c$ according to an embodiment of the present invention.

Figure 5 is a flowchart showing the calculation of the interaction between a molecular species (e.g. a molecule) and its surrounding cage in an embodiment of the present invention.

Figure 6 shows an enrichment plot of a high-throughput screening test performed using descriptors according to an embodiment of the present invention.

Figure 7 illustrates the required steps to convert a protein pocket into a three-dimensional object from which a descriptor can be obtained according to an embodiment of the present invention.

Figure 8 illustrates the representation of three examples of descriptors according to a specific example of the present invention.

Figure 9 illustrates the results of a three-dimensional quantitative structure-activity relationship (3D-QSAR) performed on descriptors according to an embodiment of the present invention.

Figure 10 illustrates the result of a three-dimensional quantitative structure-activity relationship (3D-QSAR) performed on descriptors according to an embodiment of the present invention.

Fig. 11 is an example of a computer system that may be used with the present invention.

Definitions

As used herein and unless stated otherwise, the terms "molecular species" refers to molecules of any size including macromolecules and polymers. It refers as well to inorganic molecules, organic molecules including biomolecules such as biopolymers that include proteins and polynucleic acids. It further refers to any assembly of molecules such as supramolecules, supermolecules and the like.

Detailed description of the invention

The present invention will be described with reference to certain drawings and to certain embodiments but this description is by way of example only.

In a first embodiment, the present invention relates to a computer-based method of generating a descriptor, i.e. a string of interaction values $V_i$, of a three-dimensional object represented by a set of coordinates. The method comprises the step of attributing one or more properties and a value $V_b$ for each of these one or more properties at chosen coordinates within the set of coordinates of the three-dimensional object. In this method, the following steps are performed for each of a set of one or more cages having each a three-dimensional shape on the surface of which a set of points are positioned, one or more properties and a value $V_c$ for each of said one or more properties being attributed to each of the points:

(i) enclosing entirely the three-dimensional object in a cage,
(ii) for each property, while keeping the three-dimensional object entirely enclosed in the cage by varying one or more dimensions of the cage, minimizing the interaction value $V_i$ resulting from the interaction between the three

dimensional object and the cage by changing the relative orientation between the three-dimensional object and the cage, and

(iii) assigning each of the obtained minimized interaction values $V_i$ to a distinct position in the descriptor.

As an optional feature, the value $V_c$ attributed to each of the points positioned on each cage is limited to only a few values e.g. either +1, 0 or -1, preferably +1 or -1. This feature has the advantage to permits fast computation. This also enables more easily the sum of the values $V_c$ for each cage to be zero, which is another optional feature of the present invention having the advantage to simulate a realistic globally neutral environment

As another optional feature, for the minimizing of the interaction value $V_i$, the dimensions of the cage are selected so that at least two positions on the object are closer, i.e. not farther away than 1 nm to the cage, preferably 0.3 nm to the cage. This feature is advantageous because it permits to calculate the descriptors of the present invention in a standardized and reproducible way.

As another optional feature, the set of points positioned on each cage comprises between four and twelve points. This is advantageous because a higher number of points would reduce the calculation speed without improving substantially the fidelity of the translation of the structure and properties of the three-dimensional object into the descriptor.

As another optional feature, at least one of the cages is stereospecific. This is advantageous because it permits to produce descriptors which keep information about the stereospecificity of the molecular species from which it is derived.

As another optional feature, the one or more cages are selected from cuboid cages on the surface of which said set of points are one of:

a) four points occupying half of the corners of each face of said cuboid cage, or
b) six points occupying the center of each face of said cuboid cage, or
c) eight points occupying all corners of said cuboid cage, or
d) twelve points occupying the middle of each edge of said cuboid cage.

Those type of cages and points distributions are advantageous because of their symmetry and their simplicity. This permits faster computational processes.

As another optional feature, the one or more cages are four or more cages. This is advantageous because the use of four or more cages improves significantly the fidelity with which the descriptor translates the structure and the properties of the three-dimensional object.

As another optional feature, each value $V_b$ is normalized before to perform step (ii). This is advantageous because normalization of the $V_b$ values improves significantly the fidelity with which small differences in the property distributions of the three-dimensional object are reflected in the calculated interaction values $V_i$.

As an optional feature, at least one of the one or more cages used in the first embodiment of the present invention is a cuboid. This is advantageous because cuboid cages lead to faster calculation and to descriptors translating with good fidelity the three-dimensional shape and the properties of the three-dimensional objects they describe.

As another optional feature, the three-dimensional object of the first embodiment of the present invention is a three-dimensional configuration of a molecular species (which can be determined either via a computer simulation, via one or more laboratory analysis means or via a combination of computer simulation and one or more laboratory analysis means) and the chosen coordinates are chosen atomic positions. This is advantageous since many applications of the use of descriptors are in the field of molecular sciences such as biology or chemistry.

As another optional feature, the chosen atomic positions of the three-dimensional configuration of the molecular species of the first embodiment of the present invention are all the atomic positions of the three-dimensional configuration of the molecular species. This is advantageous because it permits to translate into a descriptor the three-dimensional structure and the properties of the molecular species with the highest fidelity.

As another optional feature, the conformation of the three-dimensional configuration of the molecular species is varied after step (i) and before step (iii) of the first embodiment of the present invention so as to minimize, in the case of the first embodiment of the present invention, the calculated interaction value $V_i$ resulting from the interaction between the three-dimensional configuration of the molecular species and the cage. This is advantageous because it permits to increase the reproducibility of the descriptor obtained.

As another optional feature of the present invention, the three-dimensional object is a surface obtained from a biomolecule pocket, e.g. from a protein or from the glycosylation of a protein or from any substance with an antigenic determinant, by a method comprising the steps of:

a) filling the biomolecule, e.g. protein, pocket with a set of one or more spheres, and
b) generating a surface around this set of one or more spheres.

The translation of a biomolcule, e.g. protein pocket, such as e.g. an active site into a descriptor according to

an embodiment of the present invention is advantageous because it permits to classify those biomolecule, e.g. protein pockets according to the physicochemical properties of their active sites, or to evaluate ligand binding by evaluating the similarity between the respective descriptors of both the ligand and the biomolecule, e.g. protein pocket.

Another embodiment of the present invention relates to the assessing of the similarity between a reference three-dimensional object and a test three-dimensional object by calculating the similarity between the corresponding descriptors generated according to any embodiment of the present invention.

A further embodiment of the present invention relates to a method of generating a three-dimensional quantitative structure activity relationship (3D-QSAR) of a series of molecular species comprising the steps of :

a) obtaining a three dimensional configuration for each of the molecular species,
b) generating a descriptor, i.e. a string of interaction values, for each three-dimensional configurations according to any embodiment of the present invention,
c) associating each of these descriptor to a measured biological, e.g. therapeutic, activity,
d) defining a plurality of equations, each equation corresponding to one molecular species of the series, wherein in each equation the measured biological activity of the corresponding molecular species is set equal to a weighted linear combination of the interaction values, the weighted linear combination being weighted by unknown coefficients and the plurality of equations forming a system of equations, and
e) finding an at least approximate solution to the system of equations, the solution being the set of coefficients which come closest to making each equation true.

In an embodiment, the present invention relates to a computer-based method of generating a descriptor of a three-dimensional object. The descriptor of the present invention has been given the name Spectrophore™. The present invention includes several different types of descriptor, e.g. "protein descriptor" or "ligand descriptor". The present invention is particularly useful when applied to objects that are interesting to compare to each other (e.g. because they have an activity at least partly related to their structure or for any other reason). Typical although non-limitative examples are solid surfaces (such as but not limited to catalytic surfaces), molecular species (such as but not limited to biologically active molecular species or catalytic molecular species) or interior surfaces of protein pockets among others. In the rest of the present description, the present invention will be described mainly with reference to objects which are biologically active molecular species.

The descriptor generated by the method of the present invention is represented as a string of interaction values $V_i$ of the form ($V_{i1}$, $V_{i2}$, ...$V_{in}$) with interaction values $V_{il}$ to $V_{in}$ being all real numbers. The total number of points in the descriptor (NS) is calculated from the number of atomic properties (NP) and the number of cages (NC) (Equation 1):

$$NS = NP \times NC \qquad \text{(Equation 1)}$$

The order occupied by the different interaction values $V_i$ within the descriptor is not a limiting feature of the present invention. Any order can be used. An example of order is a grouping of the interaction values $V_i$ within the descriptor by property type, followed by cage type. The following table illustrates this for a descriptor according to the present invention calculated using three properties and four different cages (the length of the descriptor in this example is 3 x 4 = 12 interaction values):

| **Descriptor position** | **1** | **2** | **3** | **4** | **5** | **6** | **7** | **8** | **9** | **10** | **11** | **12** |
|---|---|---|---|---|---|---|---|---|---|---|---|---|
| Cage | 1 | 2 | 3 | 4 | 1 | 2 | 3 | 4 | 1 | 2 | 3 | 4 |
| Property | 1 | 1 | 1 | 1 | 2 | 2 | 2 | 2 | 3 | 3 | 3 | 3 |

The position of each interaction value within the descriptor can be calculated from Equation 2:

$$n = c + \left(\left(p-1\right) \times NC\right) \qquad \text{(Equation 2)}$$

with n the position of the interaction value calculated from the *c*'th cage and the p'th atomic property, and NC the total number of cages.

Alternatively, *c* and *p* can be deduced from the position *n* using Equations 3 and 4:

$$p = 1 + floor\left(\frac{n-1}{NC}\right) \quad \text{(Equation 3)}$$

$$c = n - \left(NC \times floor\left(\frac{n-1}{NC}\right)\right) \quad \text{(Equation 4)}$$

with $floor\left(\frac{n-1}{NC}\right)$ in Equations 3 and 4 being a function returning the largest integer value that is less than or equal to $\left(\frac{n-1}{NC}\right)$.

In an embodiment of the present invention, the descriptor of the three-dimensional object is generated by a computer-based method including the following steps:

When the three-dimensional object is a molecular species, the first step is to determine a three-dimensional configuration and a set of coordinates, i.e. a set of atomic positions for this molecular species via a computer simulation, one or more laboratory analysis means or via a combination of computer simulation and one or more laboratory analysis means. For instance, the molecular conformations can be generated by using programs such as but not limited to CORINA or Omega, derived experimentally by using methods such as but not limited to X-ray, infra-red spectroscopy (IR) or nuclear magnetic resonance (NMR) techniques or modelled according to a pharmacophoric pattern (i.e. a three-dimensional distribution of chemical functional groups or classes which are thought to be responsible for a specific pharmacological activity) or hypothesis.

The second step consists in the generation and the attribution of one or more properties and a value $V_b$ for each of these properties at chosen positions on the surface of the three-dimensional object. When the three-dimensional object is a molecular species, the chosen positions are atomic positions of the set of atomic positions determined in the first step of the method. Preferably, the chosen atomic positions are all the atomic positions of the molecular species.

The nature of the property is not a limiting feature of the present invention and any kind of property can be used. Examples of properties usable in the present invention include but are not limited to : optical properties (such as but not limited to color, absorption, transmission, index of refraction, scattering, luminescence intensity or color and the likes), mechanical properties (such as but not limited to pressure, hardness (e.g. micro-hardness), and the likes), electrical properties (e.g. conductivity), magnetical properties (e.g. magnetic susceptibility), thermal properties (e.g. temperature), shape indices and the likes. Shape indices can be generated by calculating, for each chosen position, the position's deviation from the average radius of the three-dimensional object. This can be performed in the following way:

1. Determining the three-dimensional object centre of geometry (COG);
2. Calculating the distance between each position and the three-dimensional object COG;
3. Calculating the average radius of the three-dimensional object by averaging all the three-dimensional object distances (determined in step 2);
4. Calculating the differences between the average radius of the three-dimensional object (as calculated in step 3) and the distance of each position to the COG (as calculated in step 2);
5. Normalising the values obtained in step 4 by dividing with the average radius of the three-dimensional object (as calculated in step 3). This is the shape index.

In the case when the three-dimensional object is a molecular species, suitable properties includes atomic properties such as but not limited to atomic partial charges, atomic lipophilicities, atomic softnesses, atomic hardnesses, electrophilicities, atomic shape indices and the likes. Those properties can be calculated in many ways. The exact origin of the calculation method is not critical for the present invention. For instance, atomic partial charges, softnesses, hardnesses, and electrophilicities can be calculated by using an EEM-based approach, as described by Bultinck et al. (2002, J. Phys. Chem. A. 106, 7895-7901; 2003, J. Chem. Inf. Comput. Sci. 43, 422-428). Atomic lipophilicities can be calculated and assigned using a rule-based method as described by many research groups (see e.g. Heiden et al., 1993, J. Comput. Aided Mol. Des. 7, 503-514; Mannhold et al., 1995, J. Pharm. Sci. 84, 1410-1419; Gaillard et al., 1994, J. Comput. Aided Mol. Des. 8, 83 - 96). Atomic shape indices can be generated in a similar way as described above for shape indices by calculating, for each atom, the atom's deviation from the average molecular radius. This can be

performed in the following way:

1. Determining the molecular centre of geometry (COG);
2. Calculating the distance between each atom and the molecular COG;
3. Calculating the average molecular radius by averaging all the atomic distances (determined in step 2);
4. Calculating the differences between the average molecular radius (as calculated in step 3) and the distance of each atom to the COG (as calculated in step 2);
5. Normalising the values obtained in step 4 by dividing with the average molecular radius (as calculated in step 3). This is the atomic shape index.

The presented list of properties should not be considered as being exhaustive. Any property (e.g. atomic property) might be used as input to calculate descriptors. Other examples of such properties include topological polar surface areas (Ertl et al., 2000, J. Med. Chem. 43, 3714 - 3717), hydrogen bonding potentials (Rey et al., 2001, J. Mol. Graph. Model. 19, 521-535), 'E-state'-indices (Kier & Hall, 1990, Pharm. Res. 7, 801-807 and Hall & Kier, 1995, J. Chem. Inf. Comput. Sci. 35, 1039-1045), number of connected atoms or connected bonds, among others.

The third step consists in enclosing entirely the three-dimensional object (e.g. the molecular species) in a first cage of a set of one or more cages. The number of cages can be any number above or equal to one, preferably above or equal to four. These cages have a three-dimensional shape. The three-dimensional shape of each cage can be any shape such as but not limited to polyhedra (such as but not limited to pyramids (e.g. tetrahedron), polygonal prisms (e.g. cuboids), polygonal antiprisms, and the likes), ellipsoids (e.g. sphere), cones, and the likes including truncated versions thereof. A typical example of three-dimensional shape of a cage that can be used in the present invention is a cuboid cage. On the surface of this shape, a set of points are positioned. One or more properties and values $V_c$ for each of said one or more properties are attributed to each of these points. These properties must be the same as the properties attributed to the chosen positions of the three-dimensional objects. $V_c$ are either reals or integers and can be either positive numbers or negative numbers. For instance, $V_c$ can be selected from +1, 0 or - 1. As another example, $V_c$ can be selected between +1 and -1. The sum of all the property values of a cage can be either zero or non zero. Preferably, it is zero. The number of points can be any number equal or superior to four, preferably between four and twelve. A very large number (theoretically infinite) of spatial arrangements of the points on the surface of the cages are possible.

Figure 4 illustrates examples of spatial arrangement according to a particular embodiment of the present invention, in the case of a cuboid cage.

At the top of Figure 4, a cuboid cage (a) is displayed. Four points marked p1 to p4 occupy half of the corners (1) of each face (2) of the cuboid cage (a) (quadrupole). Directly below cage (a), a cuboid cage (b) is displayed. Six points marked p1 to p6 occupy the center of each face (2) of the cuboid cage (b) (hexapole). Directly below cage (b), a cuboid cage (c) is displayed. eight points marked p1 to p8 occupy all corners (1) of the cuboid cage (c) (octapole). At the bottom of Figure 4, a cuboid cage (d) is displayed. Twelve points marked p1 to p12 occupy the middle of each edge (3) of the cuboid cage (d) (dodecapole). The table presented below shows a series of 58 cages when for instance four different arrangements (quadrupole, hexapole, octapole and dodecapole as defined in figure 4) and two property values (+1 and -1) are used.

| **Type** | **Property value $V_c$ at position** | | | | | | | | | | | | **Stereo-specific?** |
|---|---|---|---|---|---|---|---|---|---|---|---|---|---|
| | **1** | **2** | **3** | **4** | **5** | **6** | **7** | **8** | **9** | **10** | **11** | **12** | |
| Quadrupole | +1 | +1 | -1 | -1 | | | | | | | | | No |
| Hexapole | +1 | +1 | +1 | -1 | -1 | -1 | | | | | | | No |
| Hexapole | +1 | +1 | -1 | -1 | +1 | -1 | | | | | | | No |
| Octapole | +1 | -1 | +1 | -1 | | +1 | -1 | +1 | | | | | No |
| Octapole | +1 | -1 | +1 | +1 | -1 | -1 | -1 | +1 | | | | | No |
| Octapole | +1 | +1 | -1 | -1 | -1 | -1 | +1 | +1 | | | | | No |
| Octapole | +1 | +1 | -1 | -1 | +1 | +1 | -1 | -1 | | | | | No |
| Octapole | -1 | +1 | +1 | +1 | +1 | -1 | -1 | -1 | | | | | No |
| Octapole | +1 | -1 | +1 | +1 | -1 | -1 | +1 | -1 | | | | | Yes |
| Octapole | +1 | -1 | -1 | +1 | -1 | -1 | +1 | +1 | | | | | Yes |
| Dodecapole | +1 | +1 | -1 | -1 | -1 | +1 | +1 | -1 | -1 | -1 | +1 | +1 | No |

(continued)

| Type | Property value $V_c$ at position | | | | | | | | | | | | Stereo-specific? |
|---|---|---|---|---|---|---|---|---|---|---|---|---|---|
| | 1 | 2 | 3 | 4 | 5 | 6 | 7 | 8 | 9 | 10 | 11 | 12 | |
| Dodccapole | +1 | +1 | -1 | -1 | +1 | -1 | -1 | +1 | -1 | -1 | +1 | +1 | No |
| Dodecapole | +1 | +1 | +1 | -1 | -1 | -1 | -1 | -1 | -1 | +1 | +1 | +1 | No |
| Dodecapole | +1 | +1 | +1 | -1 | -1 | -1 | -1 | -1 | +1 | +1 | -1 | +1 | No |
| Dodecapole | +1 | +1 | +1 | -1 | -1 | +1 | -1 | +1 | -1 | -1 | +1 | -1 | No |
| Dodecapole | +1 | +1 | +1 | -1 | +1 | -1 | +1 | -1 | -1 | -1 | +1 | -1 | No |
| Dodecapole | +1 | +1 | +1 | -1 | +1 | -1 | +1 | -1 | +1 | -1 | -1 | -1 | No |
| Dodecapole | +1 | +1 | +1 | +1 | -1 | -1 | -1 | -1 | +1 | -1 | +1 | -1 | No |
| Dodecapole | +1 | +1 | +1 | +1 | -1 | -1 | -1 | -1 | +1 | +1 | -1 | -1 | No |
| Dodecapole | +1 | +1 | +1 | +1 | +1 | -1 | -1 | +1 | -1 | -1 | -1 | -1 | No |
| Dodecapole | +1 | +1 | +1 | +1 | +1 | +1 | -1 | -1 | -1 | -1 | -1 | -1 | No |
| Dodecapole | +1 | +1 | +1 | -1 | -1 | +1 | -1 | -1 | -1 | +1 | -1 | +1 | No |
| Dodecapole | +1 | +1 | -1 | -1 | -1 | -1 | +1 | +1 | -1 | +1 | +1 | -1 | Yes |
| Dodecapole | +1 | +1 | +1 | -1 | -1 | -1 | -1 | -1 | +1 | -1 | +1 | +1 | Yes |
| Dodecapole | +1 | +1 | +1 | -1 | -1 | -1 | -1 | +1 | -1 | +1 | +1 | -1 | Yes |
| Dodecapole | +1 | +1 | +1 | -1 | -1 | -1 | +1 | -1 | -1 | +1 | -1 | +1 | Yes |
| Dodecapole | +1 | +1 | +1 | -1 | -1 | -1 | +1 | -1 | -1 | +1 | +1 | -1 | Yes |
| Dodecapole | +1 | +1 | +1 | -1 | -1 | -1 | +1 | -1 | +1 | -1 | +1 | -1 | Yes |
| Dodecapole | +1 | +1 | +1 | -1 | -1 | -1 | +1 | -1 | +1 | +1 | -1 | -1 | Yes |
| Dodecapole | +1 | +1 | +1 | -1 | -1 | -1 | +1 | +1 | -1 | +1 | -1 | -1 | Yes |
| Dodecapole | +1 | +1 | +1 | -1 | -1 | -1 | +1 | +1 | +1 | -1 | -1 | -1 | Yes |
| Dodecapole | +1 | +1 | +1 | -1 | -1 | +1 | +1 | -1 | -1 | -1 | -1 | +1 | Yes |
| Dodecapole | +1 | +1 | +1 | -1 | -1 | +1 | +1 | -1 | -1 | -1 | +1 | -1 | Yes |
| Dodecapole | +1 | +1 | +1 | -1 | -1 | +1 | +1 | -1 | -1 | +1 | -1 | -1 | Yes |
| Dodecapole | +1 | +1 | +1 | -1 | -1 | +1 | +1 | +1 | -1 | -1 | -1 | -1 | Yes |
| Dodecapole | +1 | +1 | +1 | -1 | +1 | -1 | -1 | +1 | +1 | -1 | -1 | -1 | Yes |
| Dodecapole | +1 | +1 | +1 | -1 | +1 | -1 | +1 | -1 | -1 | -1 | -1 | +1 | Yes |
| Dodecapole | +1 | +1 | +1 | -1 | +1 | -1 | +1 | +1 | -1 | -1 | -1 | -1 | Yes |
| Dodecapole | +1 | +1 | +1 | +1 | +1 | -1 | -1 | -1 | -1 | -1 | -1 | +1 | Yes |
| Dodecapole | +1 | +1 | +1 | +1 | +1 | -1 | -1 | -1 | -1 | -1 | +1 | -1 | Yes |
| Dodecapole | +1 | +1 | -1 | -1 | +1 | -1 | +1 | -1 | +1 | -1 | -1 | +1 | Yes |
| Dodecapole | +1 | +1 | +1 | -1 | -1 | -1 | -1 | -1 | +1 | +1 | +1 | -1 | Yes |
| Dodecapole | +1 | +1 | +1 | -1 | -1 | +1 | -1 | -1 | -1 | -1 | +1 | +1 | Yes |
| Dodecapole | +1 | +1 | +1 | -1 | +1 | -1 | -1 | -1 | -1 | +1 | -1 | +1 | Yes |
| Dodecapole | +1 | +1 | +1 | -1 | +1 | -1 | -1 | -1 | -1 | -1 | +1 | +1 | Yes |
| Dodecapole | +1 | +1 | +1 | -1 | +1 | -1 | -1 | -1 | +1 | -1 | +1 | -1 | Yes |
| Dodecapole | +1 | +1 | +1 | -1 | +1 | -1 | -1 | -1 | +1 | -1 | -1 | +1 | Yes |
| Dodecapole | +1 | +1 | +1 | -1 | +1 | +1 | -1 | -1 | -1 | -1 | -1 | +1 | Yes |

(continued)

| Type | Property value $V_c$ at position | | | | | | | | | | | | Stereo-specific? |
|---|---|---|---|---|---|---|---|---|---|---|---|---|---|
| | 1 | 2 | 3 | 4 | 5 | 6 | 7 | 8 | 9 | 10 | 11 | 12 | |
| Dodecapole | +1 | +1 | +1 | -1 | +1 | +1 | -1 | -1 | +1 | -1 | -1 | -1 | Yes |
| Dodecapole | +1 | +1 | +1 | -1 | +1 | -1 | -1 | +1 | -1 | +1 | -1 | -1 | Yes |
| Dodecapole | +1 | +1 | +1 | -1 | +1 | -1 | -1 | +1 | -1 | -1 | +1 | -1 | Yes |
| Dodecapole | +1 | +1 | +1 | -1 | +1 | -1 | -1 | +1 | -1 | -1 | -1 | +1 | Yes |
| Dodecapole | +1 | +1 | +1 | -1 | +1 | +1 | -1 | +1 | -1 | -1 | -1 | -1 | Yes |
| Dodecapole | +1 | +1 | +1 | -1 | +1 | +1 | -1 | -1 | -1 | -1 | +1 | -1 | Yes |
| Dodecapole | +1 | +1 | +1 | -1 | +1 | -1 | +1 | -1 | -1 | +1 | -1 | -1 | Yes |
| Dodecapole | +1 | +1 | +1 | -1 | +1 | +1 | +1 | -1 | -1 | -1 | -1 | -1 | Yes |
| Dodecapole | +1 | +1 | +1 | +1 | +1 | -1 | -1 | -1 | +1 | -1 | -1 | -1 | Yes |
| Dodecapole | +1 | +1 | +1 | +1 | +1 | -1 | -1 | -1 | -1 | +1 | -1 | -1 | Yes |

Depending on the values $V_c$ attributed to each of those points, some of these spatial arrangements are stereo specific (*i.e.* there is a difference between the stereo specific cage and its mirror image), while other arrangements are non-stereo specific. The position and the orientation of the cage are generated in such a way that the cage encloses the three-dimensional object (e.g. the molecular species) entirely. In the example of the cages in the table hereabove, which are all cuboid cages, a number of constraint can be imposed.

A first possibility is to consider the x-, y-, and z-edges of the cuboid frame as being independent of each other, and are defined by the x-, y-, and z-extends of the molecular species. Another possibility is to consider that two out of three edges of the cuboid frame are correlated to each other, while the third edge is uncorelated. For instance, both edges could be kept at the same size or one could be kept twice as long as the other. Another possibility is to keep all three edges of the cuboid frame correlated to each other. One example is to keep all edges at the same size (the cuboid would here be a cube). Preferably, all edges are kept independent of each others. Preferably and independently of the shape of the cage selected, the dimensions of the cage are varied in order to minimise the distance between the cage and the three-dimensional object, more preferably, the dimensions of the cage are selected so that at least two positions on the object are not farther away, i.e. are closer, than 1 nm to the cage, most preferably 0.3 nm to the cage.

The fourth step consists in the calculation of the interaction between the three-dimensional object (e.g. the molecular species) and the cage. This will here be illustrated for a molecular species. The interaction between the molecular species and the enclosing cage can be estimated using standard equations well known to those in the art. One such equation is that of a typical coulombic interaction energy (Equation 5):-

$$IE = \sum_{b=1}^{nAtoms} \sum_{c=1}^{nPoints} \frac{f(v_b)g(v_c)}{h(d_{bc})} \quad \text{(Equation 5)}$$

with *IE* the interaction energy for a particular property between a given molecular species and a given cage, *nAtoms* the total number of atoms in the given molecular species, *nPoints* the total number of points to which a property is assigned in the given cage, $v_b$ the value of the property at the *b*'th atom, $v_c$ the value of the property at the *c*'th cage point, and $d_{bc}$ the distance between atom *b* and cage point *c*. Functions *f()*, *g()*, and *h()* are mathematical transformation functions well known to those in the art. For instance, $f(x) = x$, $g(x) = x$, and $h(x) = x$. Transformations such as $h(x) = x + k$ or $h(x) = x^2 + k$ can be used as well (with k being a constant). The interaction energy *IE* is a specific example of interaction value $V_i$ among others.

Other equations to evaluate the interaction between molecular species and cage are possible, and their application depends on the results one wants to achieve. For example, it might sometimes be desirable to evaluate the field similarity as a measure for interaction between a molecular species and a cage. Field similarity calculations have for example been described by Vinter et al (1995, J. Comput.-Aided Mol. Des. 9, 297-307) and Mestres et al. (1997, J. Mol. Graph. Model. 15, 114-121).

It is also possible to normalise the property values $V_b$ of the chosen positions of the three-dimensional object

(e.g. of the atoms) prior to the evaluation of the interaction. This can be done in a number of ways, including for example by normalisation towards zero mean and unit variance, or by normalisation using a scaling factor such as for example described by Cheeseright *et al.* in WO 2004/023349:

$$f_m = 1 + \sum_{b=1}^{nAtoms} \left( \frac{v_b / v_m}{1 + \left( d_{bm} / \alpha \right)^2} \right) \qquad \textbf{(Equation 6)}$$

with $f_m$ being the scale factor to normalise atom *m*, $V_b$ and $V_m$ the property values of positions (e.g. atoms) *b* and *m*, respectively, $d_{bm}$ the distance between positions (e.g. atoms) *b* and *m*, and $\alpha$ a scale factor.

For the calculation of the descriptors, it was opted to use the lowest possible interaction energy as an estimate for the interaction between the molecular species and cage for a given property. Other ways to estimate this interaction energy can be used such as but not limited to the highest interaction energy or an interaction energy averaged over all orientations and/or conformations among others. For the purpose of minimising the interaction value $V_i$, the relative orientation between enclosing cage and molecular species has to be sampled over the entire rotational space. This is achieved by performing a full systematic search of the rotational space of the molecular species in small angular steps over the x-, y-, and z-axis, while keeping the orientation of the cage fixed. Any value for the angular steps can be chosen such as 40 degrees or below, preferably 30 degrees or below and most preferably 20 degrees or below. The lower limit for the value of the angular step is only limited by the available time and the fastness of the computer used. This because the lower this limit, the longer the required computing time for a given computer. Alternately, the molecular species can be fixed and the cage rotated. Alternative interaction energy optimisation methods, well known to those in the art, may also be used such as but not limited to Monte-Carlo optimisation, molecular dynamics, minimisation techniques such as Newton-Raphson or conjugate gradients and the likes.

Optionally, in the case of molecular species, in addition to sampling the entire rotation space, sampling of the conformational space of the molecular species may also be performed if required.

After every rotational step or conformational change, the sizes of the enclosing cages are adjusted to the new orientation or conformation of the molecular species. The entire process is showed in Figure 5.

According to an embodiment of the present invention as illustrated by Figure 5, once the interaction f(m,c) between the molecular species (e.g. molecule) and the cage has been calculated, the next step consists in storing the calculated interaction f(m,c) as $f(m,c)_{min}$. Then, the next step consists in modifying the relative orientation between the molecular species (e.g. molecule) and the cage. The next step, which is an optional step, is to modify the conformation of the molecular species (e.g. molecule). The next step is to generate new cage coordinates. The next step is to calculated the interaction f(m,c) between the molecular species (e.g. molecule) in its new orientation relatively to the cage and/or its new conformation. If f(m,c) is smaller than $f(m,c)_{min}$ f(m,c) becomes the new $f(m,c)_{min}$. As long as no convergence is achieved for the value of $f(m,c)_{min}$, the process going from the modifying of the relative orientation between the molecular species (e.g. molecule) and the cage to the comparison between $f(m,c)_{min}$ with f(m,c) is repeated. Once a convergent value for $f(m,c)_{min}$ is obtained, $f(m,c)_{min}$ is saved and can be assigned to a position in the descriptor.

The next step consists in assigning each of the minimized interaction values Vi obtained to a distinct position in the string of interaction values forming the descriptor.

The last step consists in repeating the entire process for all other cages and for all other properties.

Figure 1 summarizes the general process by which descriptors are generated according to an embodiment of the present invention. In this embodiment, the three-dimensional shape of an object represented by a set of coordinates is the starting point of the process. The first step of the process consists in assigning a set of property values $V_b$ to each coordinates of the three-dimensional object. The second step consists in the generation of the descriptor and the end point of the process is the generated descriptor itself.

According to an embodiment of the present invention as illustrated by Figure 2, the starting point of the method is a computer file containing molecular species (e.g. molecules). The first step of the method consist in reading a molecular species (e.g. a molecule) from the input file in order to extract this molecular species (e.g. molecule) from this file. The next step consists in generating the three-dimensional conformation of this molecular species (e.g. molecule). In parallel, a list of NC cages and a list of NP properties are prepared and the number of interaction values $V_i$ in the descriptor NS is calculated by multiplying NP by NC. In the next step, a first cage is selected. In the next step, a first property is selected. In the next step, a first interaction value of the descriptor is calculated from the first selected cage and the first selected property. If all properties of the list have not been selected yet for this cage, the next property on the list is selected and the next interaction value $V_i$ of the descriptor is calculated from the first selected cage and the

second selected property. This process is repeated until all properties on the list have been selected and the corresponding interaction values $V_i$ of the descriptor calculated. If all cages of the list have not been selected yet, the next cage on the list is selected and the first property on the list is selected. In the next step, the next interaction values $V_i$ of the descriptor is calculated from the second selected cage and the first selected property. If all properties of the list have not been selected yet for this cage, the next property on the list is selected and the next interaction values $V_i$ of the descriptor is calculated from the second selected cage and the second selected property. This process is repeated until all properties on the list have been have been selected and the corresponding interaction values $V_i$ of the descriptor calculated. This process is repeated until all cages on the list have been selected and the corresponding interaction values $V_i$ of the descriptor calculated. The end point of this process is the resulting descriptor containing NS interaction values $V_i$.

According to an embodiment of the method illustrated by figure 3, the starting point of the method is a molecular species (e.g. a molecule), a list of properties and a list of cages. In a first step of the method, an appropriate property and an appropriate cage are selected from their corresponding lists. In the next step of the method, a property value of the selected appropriate property is assigned to each atom of the molecular species (e.g. molecule). In the next step, the selected appropriate cage is positioned around the molecular species (e.g. molecule). In the next step, the interaction between the cage and the molecular species (e.g. molecule) is calculated and in the final step of the method the calculated interaction is stored as an interaction values $V_I$ in the descriptor.

In another embodiment of the present invention, the descriptor can be generated from the active site of a protein, or any other pocket, of which the three-dimensional structure is available. This structure could be obtained by means of protein X-ray crystallography, NMR, or a theoretical approach. The hereby generated descriptors are named 'protein descriptors". These protein descriptors can be used to classify proteins according the physicochemical properties of their active sites, or to evaluate ligand binding by evaluating the similarity between the respective descriptors of both ligand and protein.

Those descriptors are normally generated in four distinct steps:

1. Filling of the active site pocket with a set of cavity spheres;

2. Generation of a set of surface points around the set of cavity spheres;

3. Projection of the protein atomic properties onto the set of surface points;

4. Generation of a descriptor from the set of annotated surface points.

Sometimes it may be desirable to work via an intermediate step, in which the surface properties, as projected from the protein atoms, are fitted onto the set of spheres centers. This set is then used to generate a descriptor. The steps involved are in this case:

1. Filling of the active site pocket with a set of cavity spheres;

2. Generation of a set of surface points around the set of cavity spheres;

3. Projection of the protein atomic properties onto the set of surface points;

4. Reverse fitting of the properties on the surface points onto the set of cavity spheres;

5. Generation of a descriptor from the set of annotated cavity spheres.

The entire process is visually depicted in Figure 7 and outlined below.

The first step relates to the filling of the active site pocket (4) with a set of cavity spheres (5).

Many algorithms have been described to fill a cavity (4) with a set of spheres (5). The algorithm as described by Laskowski (1995, J. Mol. Graph. 13, 323-330) can be implemented *mutatis mutandis.* In this procedure, each cavity sphere (5) is placed between a pair of protein atoms (6) midway between their van der Waals surfaces and just touching each. If any neighbouring protein atoms (6) penetrate this cavity sphere (5) its radius is reduced until it just touches the intruding atom (6). If the radius of the cavity sphere (5) falls below some predetermined minimum (for instance1.5 A), it is rejected. Otherwise, the sphere (5) is accepted and saved. When all pairs of protein atoms (6) have been considered the saved cavity spheres (5) fill the protein pocket. It is not always necessary to consider all atoms of the protein. It is sometimes sufficient to consider only 2-3 layers of protein atoms around the pocket.

For a typical protein active site (4), such as the ATP-binding pocket of a protein kinase as shown in Figure 7a,

the here described sphere filling algorithm generates between 100 and 2000 sphere points (5) (Figure 7b). In Figure 7b, the sphere points are represented linked to neighbouring sphere points by lines. Those lines is a feature of the graphics program used and not a feature of the sphere filling algorithm. Each of these spheres (5) are described by the coordinates of the sphere centers and the corresponding sphere radii.

The second step relates to the generation of a set of surface points (7) around the set of cavity spheres (5).

From the set at hand of sphere points with corresponding radii, a surface is calculated that encompasses these sphere points. The surface resembles the van der Waals surface of the set of cavity spheres (5), except that crevices between the spheres (5) are smoothed over and interstices too small to accommodate the surface generation probe are eliminated. The required sphere center coordinates and radii are calculated in step 1, and surrounding protein atoms are excluded from the surface calculation. The molecular surface can for instance be calculated by the method described in Richards (1977, Ann. Rev. Biophys. Bioeng. 6, 151-176). According to Richards's definition, the molecular surface consists of two parts: contact surface and reentrant surface. The contact surface is made up of those parts of the van der Waals surface that can actually be in contact with the surface of the probe. The reentrant surface is defined by the interior-facing part of the probe when it is simultaneously in contact with more than one sphere point.

In the context of this invention, it was also found that an uniform density of surface points on the surface is critical to obtain a uniform protein property sampling in the subsequent steps 3 and 4. Surface point (7) densities for a typical surface encompassing a set of cavity sphere points (5) within a protein active site (4) can be equal or superior to 0.1 surface points (7) per squared Angstrom, preferably equal or superior to 0.5 surface points (7) per squared Angstrom. The higher limit for the surface point (7) density is only defined by the available time and the speed of the computer used. This because the higher this surface point (7) density, the longer the required computing time for a given computer.

For a typical protein active site (4) such as the ATP-binding pocket of a protein kinase, the here described surface generation algorithm generates between 1000 and 5000 surface points (7) (Figure 7c).

The third step relates to the projection of the protein atom properties onto the set of surface points (7).

In order to generate a descriptor from the set of surface points derived during step 2, each of the surface points (7) needs to be annotated with at least one or more property values. These property values are generated by projecting each of the different property values of the protein atoms onto each surface point (7), as can be done using the following equation:

$$v_p = \sum_{b=1}^{nAtoms} \frac{f(v_b)}{h(d_{b,p})} \qquad \text{(Equation 7)}$$

with $v_p$ the projected property value at surface point p, *nAtoms* the total number of atoms in the given protein, $v_b$ the value of the property at the *b*'th protein atom, and $d_{b,p}$ the distance between protein atom *b* and surface point *p.*

Functions *f()* and *h()* are mathematical transformation functions well known to those in the art. For instance, $f(x) = x$ or $-x$, and $h(x) = x$. Transformations such as $h(x) = x + k$ or $h(x) = x^2 + k$ can be used as well (with k being a constant).

The here described procedure is repeated for each surface point (7), and for each property. Depending on the desired applications of the descriptors, a number of properties and transformation functions *f(x)* can be applied:

- If the purpose of descriptors is to compare and cluster a large series of protein active sites based on their physicochemical properties, it might be sufficient to use the well-known z-scales as property values (Sandberg et al., 1998, J. Med. Chem. 41, 2481-2491). In this case it is sufficient to assign, for example, each protein Cα atom the three z-scale values according the corresponding residue classification, and to transfer these atomic z-scale values onto each of the surface points (7) using equation 7. In this example, each of the surface points (7) will have three property values assigned (corresponding to the three z-scales).
- Alternatively, the protein descriptors obtained from protein pockets can also be compared with the descriptors of ligands (ligand descriptors). This might be performed in cases where one wants to evaluate the likelihood of ligand binding to a particular binding pocket, based on the assumption that high similarity between the descriptor of a protein pocket and the descriptor of a ligand is indicative of the complementarity between the protein pocket and the ligand. In these cases, it might often be necessary to guarantee that the properties which are used to generate both types of descriptors are fully compatible. In order to achieve this, it is both necessary that properties are used that can be calculated both for protein atoms as well as ligand atoms, and that appropriate transformation functions *f(x)* are applied to the protein properties. The purpose of these functions is to transform each of the property values of the protein atoms in a compatible format with the corresponding values of the ligand atoms. For instance, the following properties and transformation functions can be implemented:

| Property | Transformation function *f(x)* |
|---|---|
| Partial charges | *x* = -*x* |
| Lipophilicity | *x* = *x* |
| Softness | *x* = *x* |
| Hardness | *x* = *x* |
| Electrophilicity | *x* = *x* |

The final step in the generation of descriptors of protein pockets (protein descriptors) is to convert the surface points (7) with corresponding property values into a descriptor. This is achieved as previously described.

An Optional step is the reverse fitting of the properties on the surface points (7) onto the set of cavity spheres (5).

Once the protein properties have been projected onto the surface points (step 3), it is possible to fit the generated surface properties onto the interior sphere centers (5) before calculating a descriptor. Although not strictly required, it is nevertheless sometimes desirable to include this step since the protein descriptor which results from these annotated sphere center points will resemble more closely to a typical ligand descriptor (ligand descriptor) in terms of its absolute values.

This reverse fitting step can be implemented for instance following the method as described by Bayly et al. (1993, J. Phys. Chem. 97, 10269-10280) among others. According to this procedure, the properties of the cavity spheres (5) are calculated by fitting these properties to reproduce the properties of the surface points. A least-squares procedure is used to fit the property value $q_j$ to each sphere cavity *j*. The calculated potential at each of the surface points is given by:

$$\hat{v}_p = \sum_{j=1}^{nSpheres} \frac{q_j}{d_{pj}} \qquad \text{(Equation 8)}$$

so that the figure-of-merit $\chi^2$ to be minimized in the least-squares procedure is defined by:

$$\chi^2 = \sum_{p=1}^{nSurfacePoints} \left(v_p - \hat{v}_p\right)^2 \qquad \text{(Equation 9)}$$

Another embodiment of the present invention relates to a method of assessing the similarity between a reference three-dimensional object and a test three-dimensional object. The reference three-dimensional object can for instance be the binding pocket of a protein, a molecular species with known (biological or catalytic) activity (e.g. a molecular species with known affinity for a target protein or with known therapeutical effect) among others. In the case of molecular species, those test three-dimensional objects can for instance be found in databases of existing or virtual molecular species. The aim of the screening of a databank is in general to find three-dimensional objects (e.g. molecular species) with similar activities (e.g. catalytic or biological activities) to that of the reference three-dimensional object. In order to assess the similarity between the reference three-dimensional object and the test three-dimensional object(s), the descriptors of both the reference and the test three-dimensional objects are calculated and compared as previously described. A descriptor based virtual high throughput screening is typically performed in five distinct steps:

1) generation of molecular conformations from the molecular species of a database;
2) generation of the descriptor corresponding to the molecular conformations generated in 1);
3) calculation of a reference descriptor;
4) similarity calculation between the reference descriptor and the test descriptors;
5) sorting the database and selection of database molecular species having a higher similarity with the reference descriptor.

Another embodiment of the present invention relates to a method of generating a three-dimensional quantitative structure activity relationship (3D-QSAR) of a series of molecular species. QSAR are mathematical relationships linking chemical structures represented in the form of descriptors and biological activity in a quantitative manner for a series of molecular species. The QSAR method of the present invention is three-dimensional because it makes use of descriptors derived from three-dimensional molecular representations of species.

The first step of the method consists in obtaining a three-dimensional configuration for each of the molecular species of the series. This can be done as previously described.

The second step of the method consists in generating a descriptor for each three-dimensional configuration. This can be done as previously described.

The third step of the method consists in associating each descriptor to a biological activity.

The fourth step of the method consists in defining a plurality of equations, each equation corresponding to one molecular species of the series, wherein in each equation, the measured biological activity of the corresponding molecular species is set equal to a weighted linear combination of said values. this weighted linear combination is weighted by unknown coefficients and the plurality of equations forms a system of equations.

The fifth step consist in finding an at least approximate solution to the system of equations. This solution is the set of coefficients coming the closest to make each equation true.

Such method embodiments as are described above may be implemented in a processing system 150 such as shown in FIG. 11. FIG. 11 shows one configuration of processing system 150 that includes at least one programmable processor 153 coupled to a memory subsystem 155 that includes at least one form of memory, e.g., RAM, ROM, and so forth. A storage subsystem 157 may be included that has at least one disk drive and/or CD-ROM drive and/or DVD drive. In some implementations, a display system, a keyboard, and a pointing device may be included as part of a user interface subsystem 159 to provide for a user to manually input information. Ports for inputting and outputting data also may be included. More elements such as network connections, interfaces to various devices, and so forth, may be included, but are not illustrated in FIG. 11. The various elements of the processing system 150 may be coupled in various ways, including via a bus subsystem 163 shown in FIG. 11 for simplicity as a single bus, but will be understood to those in the art to include a system of at least one bus. The memory of the memory subsystem 155 may at some time hold part or all (in either case shown as 161) of a set of instructions that when executed on the processing system 150 implement the step(s) of any of the method embodiments described herein. Thus, while a processing system 150 such as shown in FIG. 11 is prior art, a system that includes the instructions to implement novel aspects of the present invention is not prior art, and therefore FIG. 11 is not labelled as prior art.

It is to be noted that the processor 153 or processors may be a general purpose, or a special purpose processor, and may be for inclusion in a device, e.g., a chip that has other components that perform other functions, for example it may be an embedded processor. Also with developments such devices may be replaced by any other suitable processing engine, e.g. an FPGA. Thus, one or more aspects of the present invention can be implemented in digital electronic circuitry, or in computer hardware, firmware, software, or in combinations of them. Furthermore, aspects of the invention can be implemented in a computer program product tangibly embodied in a carrier medium carrying machine-readable code for execution by a programmable processor. Method steps of aspects of the invention may be performed by a programmable processor executing instructions to perform functions of those aspects of the invention, e.g., by operating on input data and generating output data.

Furthermore, aspects of the invention can be implemented in a computer program product tangibly embodied in a carrier medium carrying machine-readable code for execution by a programmable processor. The term "carrier medium" refers to any medium that participates in providing instructions to a processor for execution. Such a medium may take many forms, including but not limited to, non-volatile media, and transmission media. Non-volatile media includes, for example, optical or magnetic disks, such as a storage device which is part of mass storage. Volatile media includes mass storage. Volatile media includes dynamic memory such as RAM. Common forms of computer readable media include, for example a floppy disk, a flexible disk, a hard disk, magnetic tape, or any other magnetic medium, a CD-ROM, any other optical medium, punch cards, paper tapes, any other physical medium with patterns of holes, a RAM, a PROM, an EPROM, a FLASH-EPROM, any other memory chip or cartridge, a carrier wave as described hereafter, or any other medium from which a computer can read. Various forms of computer readable media may be involved in carrying one or more sequences of one or more instructions to a processor for execution. For example, the instructions may initially be carried on a magnetic disk of a remote computer. The remote computer can load the instructions into its dynamic memory and send the instructions over a telephone line using a modem. A modem local to the computer system can receive the data on the telephone line and use an infrared transmitter to convert the data to an infrared signal. An infrared detector coupled to a bus can receive the data carried in the infrared signal and place the data on the bus. The bus carries data to main memory, from which a processor retrieves and executes the instructions. The instructions received by main memory may optionally be stored on a storage device either before or after execution by a processor. The instructions can also be transmitted via a carrier wave in a network, such as a LAN, a WAN or the Internet. Transmission media can take the form of acoustic or light waves, such as those generated during radio wave and infrared data communications. Transmission media include coaxial cables, copper wire and fibre optics, including the wires that comprise a bus within a computer.

Example 1:

In this example, the protein descriptor of the active site of a cAMP-dependent protein kinase in complex with MNAMP-PNP (PDB-code: 1CDK), using z-scales as protein properties and with dodecapoles as interaction cages, has been calculated and looks as follows:

(1177.0235104, 1241.9454472, 5309.7227832, 7739.9044175, 3592.9170204, 3663.1235402, 3318.7667805, 5557.9750177, 4513.2625606, 4810.0268823, 4637.8198335, 7039.2447879, 3817.8125232, 2729.4127530, 8871.0996326, 7871.9820890, 4041.8094676, 2803.1417433, 2658.5126972, 8796.1666293, 7880.8818501, 5062.2250856, 4396.6414940, 8478.5168524, 1093.6344578, 571.7572775, 869.4288781, 2070.4729018, 2584.8578515, 2290.0349962, 1641.3678263, 1685.1768608, 1948.6745828, 3197.5941913, 3538.7587911, 2291.4695265)

This descriptor is generated from the three z-scale property values in combination with twelve non-stereospecific dodecapole cages, resulting in a total of 36 data points (12 cages times 3 properties).

The descriptor for the same cAMP-dependent protein kinase, however now in complex with MNATP (PDB-code: 1ATP), is:

(1451.0615659, 1452.3946320, 4791.0736889, 6525.2927839, 4491.6511286, 3202.0090141, 2376.0092687, 4881.4240683, 5349.9589960, 4515.2041767, 5586.4489508, 5915.8262652, 4405.5709945, 3078.4106003, 8424.1878645, 8998.8770307, 5154.5239871, 4336.7009250, 3026.1709973, 8173.1688876, 8747.2194043, 6645.3295798, 6430.0893508, 6598.1402542, 904.00873950, 548.4988372, 694.4875240, 2051.4446135, 2530.8606115, 2320.2737774, 1939.2617181, 2157.2011781, 2093.2625232, 3375.3180364, 3552.9222586 2349.7615051)

On the other hand, the generated descriptor for the ATP-binding pocket of a different protein kinase, the CDK2 protein kinase in complex with purvanalol B (PDB-code: 1 CKP), differentiate itself clearly from the descriptors of the two cAMP-dependent kinases:

(2382.6536642, 1474.7309051, 2092.6384519, 4267.5679973, 6382.3308870, 5523.3496559, 4150.7807419, 3018.0932592, 4312.2598303, 6536.9238770, 7715.0819416, 4417.0939360, 2061.3033873, 1464.6906777, 3633.7372348, 5459.4372702, 6556.4724403, 4929.9911477, 4520.3761649, 5945.4945549, 6251.8003512, 7558.1630982 8220.1978131, 7044.5718066, 504.9865912, 300.1583540, 537.9934145, 802.0223370, 1176.5070111, 1174.3539725, 1059.1203880, 1120.4337701, 1124.4183724, 1451.3004884, 1687.0289938, 1102.4849312)

The three descriptors are graphically compared in Figure 8. Figure 8 shows the similarities between the respective descriptors of 1CDK and 1ATP and the differences between those two descriptors and the descriptor corresponding to 1 CKP.

1 CDK and 1ATP are both cAMP-dependent protein kinases, but with different inhibitors bound to their active sites, while 1 CKP is the protein kinase CDK2.

Example 2

The here described example of virtual screening is based on research as published by McElroy and coworkers in 2003 (J. Med. Chem. 46, 1066-1080). In this work, the inhibition of human and murine soluble epoxide hydrolase by a large series of urea-like molecular species has been described. For the purpose of this example, ten molecular species, all with $IC_{50}$ values less or equal than 0.2 μM, were randomly selected from the publication and were subsequently divided in two sets:

- The 'actives' set. Three out of the ten molecular species were labelled as being the 'confirmed active' molecular species. These molecular species were randomly chosen from the ten selected molecular species, and were used to calculate a single reference descriptor with associated weight factors. This reference descriptor with its associated weight factors was used to search a molecular database for molecular species having similar activities.
- The database of 'unknowns'. The remaining seven molecular species were inserted in a subset of the Maybridge molecular database containing 993 drug-like molecular species of which the pharmacological activity with respect to epoxide hydrolase inhibition was unknown. The resulting database consisted therefore of in total 1000 molecular species, of which at least seven molecular species had an $IC_{50}$ value less or equal than 0.2 μM for the inhibition of human soluble epoxide hydrolase.

The chemical structures and inhibition constants of the ten molecular species are shown in the table below:

| ID | Structure | $IC_{50}$ (μm) | Set |
|---|---|---|---|
| 122 | 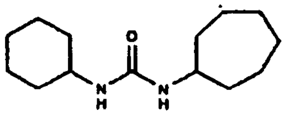 | 0.10 | 'Confirmed active' |
| 125 | 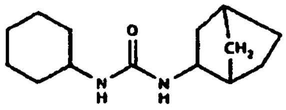 | 0.10 | 'Confirmed active' |
| 127 | 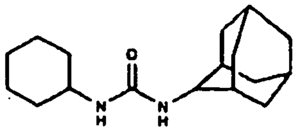 | 0.10 | 'Confirmed active' |
| 136 | 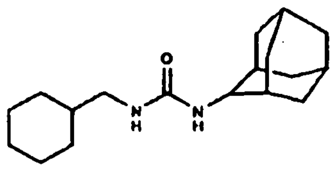 | 0.10 | 'Unknown active' |
| 141 | 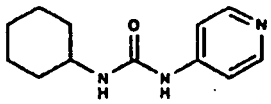 | 0.15 | 'Unknown active' |
| 143 | 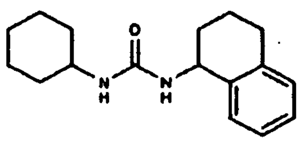 | 0.10 | 'Unknown active' |
| 149 | 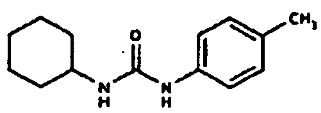 | 0.17 | 'Unknown active' |
| 153 | 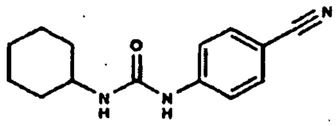 | 0.10 | 'Unknown active' |
| 155 | 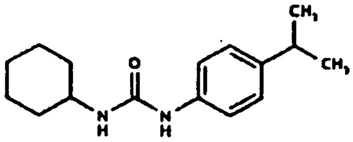 | 0.07 | 'Unknown active' |
| 163 | 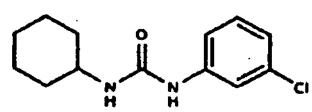 | 0.19 | 'Unknown active' |

The purpose of this example is to demonstrate the applicability of the descriptors according to the present invention with respect to the selection from databases of molecular species with well-defined pharmacological properties. Prior to the generation of descriptors, molecular conformations need to be calculated. For this purpose, conformations were calculated for each molecular species using the OMEGA program of OpenEye Scientific Software. This resulted in a single three-dimensional conformation for each molecular species. Descriptors were calculated for all the molecular

species (the three molecular species of the 'confirmed actives' set, and the 1000 database molecular species). The following atomic properties were used to calculate the descriptors:

- Shape index,
- Partial atomic charges, using the EEM-approximation (Bultinck et al., 2002, J. Phys. Chem. A. 106, 7895-7901);
- Atomic electrophilicities, using the EEM-approximation;
- Atomic softness, using the EEM-approximation;
- Atomic lipophilicities, as described by Gaillard et al., 1994, J. Comput. Aided Mol. Des. 8, 83-96.

Twelve non-stereospecific dodecapole cages were used for the calculation of the interaction values $V_i$, which resulted for each molecular species in a descriptor consisting of 60 interaction values $V_i$ [12 (number of cages) by 5 (number of properties)]. For example, the descriptor of molecular species **122** in the table hereabove is given: (1.6493547, 2.4467502, 3.0259994, 3.1405621, 2.6612200, 2.2822862, 1.7679167, 3.4061206, 3.0566774, 2.8076934, 2.6721218, 3.6771842, 1.3812835, 2.4409513, 3.3207675, 2.9868924, 1.4800005, 1.5467830, 0.4984696, 3.3188026, 3.2676320, 1.3759867, 0.9193736, 2.2404641, 9.3956598, 15.5909196, 9.5878893, 8.9463201, 9.9863238, 11.1901966, 3.5517550, 9.4718922, 10.8558505, 10.2552206, 6.7633472, 11.8095674, 31.6654207, 51.7477946, 63.6184823, 72.0496439, 41.9713097, 47.7432800, 18.1939267, 60.4060183, 41.2523642, 40.5303563, 38.2618646, 77.0991979,18.3943909, 32.5058646, 44.2222734, 39.7760980, 19.7089946, 20.5983293, 6.6380618, 44.1961070, 43.5146740, 18.3238541, 12.2431910, 29.8359995)

Next, a reference descriptor has been calculated by taking the weighed average of the three individual descriptors of molecular species **122, 125,** and **127** (the 'actives' set). This resulted in the following reference descriptor: (1.6499500, 2.4562700, 3.0806100, 3.2749400, 3.3286100, 2.5996200, 2.1886500, 3.6824700, 3.4585900, 3.1786300, 3.2799000, 3.5231800, 1.3174300, 2.3967500, 3.1889000, 3.0633700, 1.4048900, 1.5853000, 0.4906850, 3.3675900, 3.3374600, 1.6244800, 1.0346100, 2.1837400, 9.2680200, 16.2342000, 10.0758000, 9.4229200, 10.7091000, 14.7422000, 5.7789300, 13.2777000, 13.8001000, 13.7852000, 11.4665000, 12.1918000, 30.5559000, 51.4414000, 61.0996000, 78.3450000, 39.8737000, 54.7513000, 24.0170000, 59.6234000, 45.8535000, 51.1712000, 47.4466000, 80.5812000, 17.4780000, 31.8035000, 42.2614000, 40.5944000, 18.6294000, 21.0721000, 6.5280600, 44.6714000, 44.2680000, 21.5703000, 13.7544000, 28.9717000)

Calculation of the weighed average can be achieved in a number of ways. However, in the present example, the weights were taken as being the statistical F-ratios from the set of the three 'actives', on the one hand, and the molecular species within the 'unknowns' database, on the other hand. Using the statistical F-ratios as weight factors has the advantage that differences in both means and variances are taken into account. In this example, the derived weight factors were: (1.8908700, 0.9900330, 0.5853290, 1.3174900, 1.5539800, 1.8989600, 1.7088700, 1.1945000, 1.6524800, 1.9966900, 1.6752200, 1.7104300, 1.1428600, 0.2739840, 0.9337710, 1.1269600, 2.1171600, 1.6434200, 2.5066000, 1.1415000, 0.9121310, 2.4198700, 2.6895400, 2.2596800, 0.0181191, 0.5348470, 0.8856620, 1.5213600, 1.1831100, 0.4052540, 1.5956600, 1.4294900, 1.3199900, 1.2416200, 1.6402600, 1.5015700, 0.0375171, 0.1986550, 0.1295690, 0.3360140, 0.0091109 0.1698740, 0.0228813, 0.0635029, 0.0019022, 0.0030144, 0.0076395, 0.2657470, 0.0151632, 1.0090400, 0.1722870, 0.0423767, 0.7984770, 0.0820521, 1.9608100, 0.0701633, 0.3405390, 0.9287420, . 2.1138600, 0.6018120)

Next, using the reference descriptor with its associated weight factors, and the database of 1000 molecular species of unknown activity, similarities could be calculated between the reference descriptor and each of the 1000 descriptor within the database. The similarities were obtained by calculating the normalised distance between each pair of descriptors:

$$d = \sqrt{\frac{\sum_{i=1}^{n}(s_i - r_i)^2 w_i}{\sum_{i=1}^{n} s_i^2 w_i + \sum_{i=1}^{n} r_i^2 w_i}} \quad \text{(Equation 10)}$$

with d being the normalised distance, n the number of descriptor interaction values (in this example 60), $s_i$ the $i$th point of the database descriptor, $r_i$ the $i$th point of the reference descriptor, and $w_i$ the $i$th weight factor. A large similarity is indicated by a small d, and vice versa.

The last step consisted in the selection of molecular species from the database for which the calculated descriptor have a large similarity with the reference descriptor. In the context of this example, the sorted database had the seven molecular species with an $IC_{50} \leq 0.2$ μm located within its top 4%. This means that these seven molecular species,

having an $IC_{50} \leq 0.2$ μm for sHE inhibition, were all located within the top 40 of the sorted database of 1000 molecular species, or in a non-virtual world, it should have been sufficient to biochemicaly test only the top-4% of the entire ranked database to identify at least seven other molecular species having the desired sHE inhibitory activity. This is visually depicted in Figure 6. In Figure 6, the x-axis refers to the fraction of the ranked database screened and the y-axis referred to the fraction of active molecular species founded. It shows an enrichment plot of sHE virtual high-throughput screening and it demonstrates that the seven active molecular species where all located within the top-4% of a ranked database translated to a 25-fold database enrichment.

Example 3:

As an example of the application of descriptors for the generation of quantitative structure-activity relationships, the development of a blood-brain penetration model is given here. Accurate predictions of passive drug blood-brain barrier penetration, expressed as steady-state distribution profiles, are of great interest and value to the pharmaceutical industry, and as consequence a great deal of computational models have been described in the literature during the recent years. In the context of this example, a model of passive blood-brain barrier (BBB) penetration is described using the descriptors of the present invention in combination with two optimisation models. In the context of this example, both a Generalized Linear Model (GLM) and a Support Vector Machine (SVM) have been used as optimization algorithms, although other choices, such as neural networks among others, are also possible. Experimental data were obtained from the paper by Rose and coworkers (2002, J. Chem. Inf. Comput. Sci. 42, 651-666) describing more than 100 BBB distributions in combination with structural information. Sixty-six randomly chosen data points were used as a training set, and 33 data points were kept separate as an internal test data set. External validation of the model was performed using the 57 data points as described by Clark (1999).

The first two steps consist in the generation of molecular conformations and descriptors.

For each of the in total 156 molecular species, a single conformation was generated using Omega (OpenEye Scientific Software, USA). Five atomic properties were used to calculate the descriptors:

- Shape index;
- Partial atomic charges, using the EEM-approximation ;
- Atomic electrophilicities, using the EEM-approximation;
- Atomic softness, using the EEM-approximation;
- Atomic lipophilicities, as described by Gaillard *et al.*

Five non-stereospecific and one stereospecific octapole cages were used for the calculation of the interaction values, which resulted for each molecular species in a descriptor consisting of 30 interaction values (6 values per property times 5 properties).

The third step consist in the calculation and validation of a QSAR model. Although several kernels were evaluated for the SVM optimisation, a Radial Basis Function (RBF) kernel was selected as the best choice based on the obtained results for the internal test set consisting of 33 molecular species. The resulting models from the GLM and the SVM optimisation are presented in Figure 9(a) and 9(b) respectively. In Figure 9 (a) and 9(b), the abscise and ordinate labels are in log-units of the relative brain/blood concentrations. White squares are the training set; white triangles are the test set and black points are the validation set. The respective mean-squared errors (MSE) were 0.57 for the GLM and 0.41 log-units for the SVM for the internal test set. For the external validation set, the respective mean-squared errors (MSE) were 0.44 for the GLM and 0.25 log-units for the SVM. For the external validation dataset, of the 28 molecular species experimentally determined to penetrate the blood-brain barrier, 26 molecular species are predicted correctly. Alternatively, of the 29 brain-impermeable molecular species, 24 are predicted correctly.

Significance levels for the 30 parameters according the GLM model are shown in Figure 10. Figure 10 shows the significance of each of the 30 descriptor interaction values $V_i$ with respect to blood-brain barrier penetration modelling. Electrophilicity and lipophilicity are the most significant parameters for the correlation of structures with penetration data. Both electrophilicity and lipophilicity seem to be the most significant parameters for the correlation of molecular structures with BBB-penetration data, which is consistent with the widely-accepted thought that lipophilicity plays a major role in the transport of drugs throughout the blood-brain barrier.

## Claims

1. A computer-based method of generating a descriptor of a three-dimensional object, said three dimensional object being selected from the group consisting of

1) a surface obtained from a biomolecule pocket,
2) a three- dimensional configuration of a molecular species, and
3) a catalytic surface,

said three-dimensional object being represented by a set of coordinates, said descriptor being a string of interaction values $V_i$, said method comprising the step of attributing one or more properties and a value $V_b$ for each of said one or more properties at chosen coordinates within said set of coordinates of said three-dimensional object, **characterized in that** the following steps are performed for each of a set of one or more cages, said set of one or more cages implementing an artificial environment, said one or more cages having each a three-dimensional shape on the surface of which a set of points are positioned, the same one or more properties as attributed to said three-dimensional object and a value $V_c$ for each of said one or more properties being attributed to each of said points:

i) enclosing entirely said three-dimensional object in said cage,
ii) for each property, while keeping said three-dimensional object entirely enclosed in said cage by varying one or more dimensions of said cage, determining the lowest possible interaction value $V_i$ or the highest interaction value $V_i$ or an interaction value $V_i$ averaged over all orientations resulting from the interaction between said chosen coordinates of said three dimensional object and said points of said cage by sampling over the entire rotational space the relative orientation between the three-dimensional object and said cage,and
iii) assigning each of the obtained lowest possible interaction values $V_i$ or highest interaction values $V_i$ or interaction value $V_i$ averaged over all orientations to a distinct position in said descriptor.

**2.** A method according to claim 1, **characterized in that** said value $V_c$ is either +1, 0 or-1.

**3.** A method according to claim 2, **characterized in that** said value $V_c$ is either +1 or -1.

**4.** A method according to any of claims 1 to 3, **characterized in that** the sum of the values $V_c$ for each cage is zero.

**5.** A method according to any previous claims, **characterized in that** said interaction value $V_i$ is given by the following formula:

$$V_i = \sum_{b-1}^{nCoordinates} \sum_{c=1}^{nPoints} \frac{f(V_b)g(V_c)}{h(d_{bc})},$$ wherein *nCoordinates is* the total number of said chosen coordinates in said three-dimensional object, *nPoints* the total number of said points, $V_b$ the value of the property at the *b*'th coordinate, $V_c$ the value of the property at the *c*'th cage point, $d_{bc}$ the distance between coordinate *b* and cage point *c*, and *f()*, *g()*, and *h()* are mathematical transformation functions.

**6.** A method according to any preceding claims, **characterized in that** for the minimizing of the interaction value $V_i$ the dimensions of the cage are selected so that at least two positions on the object are closer than 1 nm to the cage.

**7.** A method according to any preceding claims, **characterized in that** said set of points comprises between four and twelve points.

**8.** A method according to any preceding claims, **characterized in that** at least one of said cages is stereospecific.

**9.** A method according to any preceding claims, **characterized in that** said one or more cages are selected from cuboid cages on the surface of which said set of points are:

a) four points occupying half of the corners of each face of said cuboid cage, or
b) six points occupying the center of each face of said cuboid cage, or
c) eight points occupying all corners of said cuboid cage, or
d) twelve points occupying the middle of each edge of said cuboid cage.

**10.** A method according to any preceding claims, **characterized in that** said one or more cages are four or more cages.

**11.** A method according to any preceding claims, **characterized in that** each value $V_b$ is normalized before to perform step ii).

**12.** A method according to any preceding claims, **characterized in that** at least one of said one or more cages is a cuboid.

**13.** A method according to any preceding claims, **characterized in that** said three-dimensional object is a three-dimensional configuration of a molecular species and wherein said chosen coordinates are chosen atomic positions.

**14.** A method according to any preceding claims, **characterized in that** said chosen atomic positions are all the atomic positions of said three-dimensional configuration of said molecular species.

**15.** A method according to claim 5, **characterized in that** said three dimensional object is a three- dimensional configuration of a molecular species, ncoordinates is the total number of said chosen atomic positions in said three-dimensional configuration of a molecular species, $V_b$ is the value of the property at the *b*'th atomic position, $d_{bc}$ is the distance between atom *b* and cage point *c*.

**16.** A method according to any of claims 13 to 15, **characterized in that** said three-dimensional configuration is either determined via a computer simulation, via one or more laboratory analysis means or via a combination of computer simulation and one or more laboratory analysis means.

**17.** A method according to any of claims 13 to 16, further comprising the step of optimizing the conformation of said three-dimensional configuration of said molecular species after step i) and before step iii) so as to minimize the calculated interaction value $V_i$ resulting from the interaction between said three-dimensional configuration of said molecular species and said cage.

**18.** A method according to any preceding claims for use in virtual screening of molecular databases, virtual synthesis of molecules with predefined properties, and in understanding and in deriving structure/relationships of such molecular species.

**19.** A method of assessing the similarity between a reference three-dimensional object and a test three-dimensional object by calculating the similarity between the corresponding descriptors generated by the method of any preceding claims.

**20.** A method of generating a three-dimensional quantitative structure activity relationship 3D-QSAR of a series of molecular species comprising the steps of:

a) obtaining a three dimensional configuration for each of said molecular species,
b) generating a descriptor, said descriptor being a string of interaction values, for each three-dimensional configuration by the method of any of claims 13 to 18,
c) associating each of said descriptors to a measured biological activity,
d) defining a plurality of equations, each equation corresponding to one molecular species of the series, wherein in each equation said measures biological activity of the corresponding molecular species is set equal to a weighted linear combination of said interaction values, said weighted linear combination being weighted by unknown coefficients, said plurality of equations forming a system of equations, and
e) finding an at least approximate solution to the system of equations, said solution being the set of coefficients which come closest to making each equation true.

**21.** A computer program product comprising software code for implementing any of the methods of the claims 1 to 20 when executed on a computing system.

**22.** A machine readable data carrier storing the computer program of claim 21.

**Patentansprüche**

**1.** Rechnergestütztes Verfahren zum Erzeugen eines Deskriptors eines dreidimensionalen Objektes, wobei das dreidimensionale Objekt aus der Gruppe ausgewählt wird, welche aus Folgendem besteht:

1) einer Oberfläche, welche aus einer Biomolekültasche erhalten wird,
2) einer dreidimensionalen Konfiguration einer Molekülart, und
3) einer katalytischen Oberfläche,

wobei das dreidimensionale Objekt durch eine Gruppe an Koordinaten dargestellt ist, der Deskriptor eine Folge an Wechselwirkungswerten $V_i$ ist und das Verfahren den Schritt zum Zuschreiben von einer oder mehreren Eigenschaften und eines Wertes $V_b$ für jede der einen oder mehreren Eigenschaften an ausgewählten Koordinaten innerhalb der Gruppe an Koordinaten des dreidimensionalen Objektes aufweist, **dadurch gekennzeichnet, dass** die folgenden Schritte für jeden Käfig einer Gruppe aus einem oder mehreren Käfigen durchgeführt werden, die Gruppe aus einem oder mehreren Käfigen eine künstliche Umgebung implementiert, der eine oder die mehreren Käfige jeweils eine dreidimensionale Form aufweisen, auf deren Oberfläche eine Gruppe an Punkten positioniert ist, und die gleiche Eigenschaft oder die gleichen mehreren Eigenschaften, die dem dreidimensionalen Gegenstand zugeschrieben wurde(n), und ein Wert $V_c$ für jede der einen oder mehreren Eigenschaften jedem der Punkte zugeschrieben wird:

i) völliges Umgeben des dreidimensionalen Objektes mit dem Käfig,
ii) während das völlige Umgeben des dreidimensionalen Objektes mit dem Käfig durch Verändern eines oder mehrerer Maße des Käfigs beibehalten wird, Bestimmen des geringstmöglichen Wechselwirkungswertes $V_i$, höchsten Wechselwirkungswertes $V_i$ oder eines Wechselwirkungswertes $V_i$, dessen Mittelwert aus allen Orientierungen gebildet wurde, welche sich aus der Wechselwirkung zwischen den ausgewählten Koordinaten des dreidimensionalen Objektes und den Punkten des Käfigs durch Abtasten der relativen Orientierung zwischen dem dreidimensionalen Objekt und dem Käfig über den gesamten Rotationsraum ergeben, für jede Eigenschaft, und
iii) Zuordnen aller erhaltenen geringstmöglichen Wechselwirkungswerte $V_i$, höchsten Wechselwirkungswerte $V_i$ oder des erhaltenen Wechselwirkungswertes $V_i$, dessen Mittelwert aus allen Orientierungen gebildet wurde, einer bestimmten Position im Deskriptor.

**2.** Verfahren nach Anspruch 1, **dadurch gekennzeichnet, dass** der Wert $V_c$ entweder +1, 0 oder -1 beträgt.

**3.** Verfahren nach Anspruch 2, **dadurch gekennzeichnet, dass** der Wert $V_c$ entweder +1 oder -1 beträgt.

**4.** Verfahren nach einem der Ansprüche 1 bis 3, **dadurch gekennzeichnet, dass** die Summe der Werte $V_c$ für jeden Käfig null beträgt.

**5.** Verfahren nach einem der vorangehenden Ansprüche, **dadurch gekennzeichnet, dass** der Wechselwirkungswert $V_i$ durch die folgende Formel gegeben ist:

$$V_i = \sum_{b=1}^{nCoordinates} \sum_{c=1}^{nPoints} \frac{f(V_b)g(V_c)}{h(d_{bc})},$$

wobei *nCoordinates* die Gesamtanzahl an ausgewählten Koordinaten im dreidimensionalen Objekt ist, *nPoints* die Gesamtanzahl an Punkten ist, $V_b$ der Wert der Eigenschaft an der b-ten Koordinate ist, $V_c$ der Wert der Eigenschaft an der *c*-ten Koordinate ist, $d_{bc}$ der Abstand zwischen der Koordinate *b* und dem Käfigpunkt c ist und *f()* , *g()* und *h()* die mathematischen Transformationsfunktionen sind.

**6.** Verfahren nach einem der vorangehenden Ansprüche, **dadurch gekennzeichnet, dass** zum Minimieren des Wechselwirkungswertes $V_i$ die Maße des Käfigs derart ausgewählt werden, dass sich zumindest zwei Positionen auf dem Objekt näher als 1nm am Käfig befinden.

**7.** Verfahren nach einem der vorangehenden Ansprüche, **dadurch gekennzeichnet, dass** die Gruppe an Punkten zwischen vier und zwölf Punkte aufweist.

**8.** Verfahren nach einem der vorangehenden Ansprüche, **dadurch gekennzeichnet, dass** zumindest einer der Käfige stereotypisch ist.

**9.** Verfahren nach einem der vorangehenden Ansprüche, **dadurch gekennzeichnet, dass** der eine oder die mehreren Käfige aus quaderförmigen Käfigen ausgewählt werden, auf deren Oberfläche die Gruppe an Punkten Folgende sind:

a) vier Punkte, welche die Hälfte der Ecken jeder Fläche des quaderförmigen Käfigs besetzen, oder
b) sechs Punkte, welche die Mitte jeder Fläche des quaderförmigen Käfigs besetzen, oder
c) acht Punkte, welche alle Ecken des quaderförmigen Käfigs besetzen, oder
d) zwölf Punkte, welche die Mitte jeder Kante des quaderförmigen Käfigs besetzen.

**10.** Verfahren nach einem der vorangehenden Ansprüche, **dadurch gekennzeichnet, dass** der eine oder die mehreren Käfige vier oder mehr Käfige sind.

**11.** Verfahren nach einem der vorangehenden Ansprüche, **dadurch gekennzeichnet, dass** jeder Wert $V_b$ vor dem Durchführen des Schrittes ii) normalisiert wird.

**12.** Verfahren nach einem der vorangehenden Ansprüche, **dadurch gekennzeichnet, dass** zumindest einer des einen oder der mehreren Käfige ein Quader ist.

**13.** Verfahren nach einem der vorangehenden Ansprüche, **dadurch gekennzeichnet, dass** das dreidimensionale Objekt eine dreidimensionale Konfiguration einer Molekülart ist, und wobei die ausgewählten Koordinaten ausgewählte Atompositionen sind.

**14.** Verfahren nach einem der vorangehenden Ansprüche, **dadurch gekennzeichnet, dass** die ausgewählten Atompositionen alle Atompositionen der dreidimensionalen Konfiguration der Molekülart sind.

**15.** Verfahren nach Anspruch 5, **dadurch gekennzeichnet, dass** das dreidimensionale Objekt eine dreidimensionale Konfiguration einer Molekülart ist, *nCoordinates* die Gesamtanzahl an ausgewählten Atompositionen in der dreidimensionalen Konfiguration einer Molekülart ist, $V_b$ der Wert der Eigenschaft an der *b*-ten Atomposition ist und $d_{bc}$ der Abstand zwischen dem Atom *b* und dem Käfigpunkt *c* ist.

**16.** Verfahren nach einem der Ansprüche 13 bis 15, **dadurch gekennzeichnet, dass** die dreidimensionale Konfiguration entweder über eine Computersimulation, über eine oder mehrere Laboranalyseeinrichtungen oder über eine Kombination aus der Computersimulation und der einen oder mehreren Laboranalyseeinrichtungen bestimmt wird.

**17.** Verfahren nach einem der Ansprüche 13 bis 16, welches zudem den Schritt zum Optimieren der Konformation der dreidimensionalen Konfiguration des Molekülart nach dem Schritt i) und vor dem Schritt iii) aufweist, um den berechneten Wechselwirkungswert $V_i$ zu minimieren, welcher sich aus der Wechselwirkung zwischen der dreidimensionalen Konfiguration der Molekülart und dem Käfig ergibt.

**18.** Verfahren nach einem der vorangehenden Ansprüche zur Verwendung beim virtuellen Screening der Moleküldatenbanken, bei der virtuellen Synthese von Molekülen mit vorbestimmten Eigenschaften und beim Verstehen und Ableiten der Struktur/Beziehungen solcher Molekülarten.

**19.** Verfahren zum Beurteilen der Ähnlichkeit zwischen einem dreidimensionalen Bezugsobjekt und einem dreidimensionalen Prüfobjekt durch Berechnen der Ähnlichkeit zwischen den entsprechenden Deskriptoren, welche durch das Verfahren nach einem der vorangehenden Ansprüche erzeugt wurden.

**20.** Verfahren zum Erzeugen einer dreidimensionalen quantitativen Struktur-Aktivitäts-Beziehung, 3D-QSAR, einer Reihe an Molekülarten, welches die folgenden Schritte aufweist:

a) Erhalten einer dreidimensionalen Konfiguration für alle Molekülarten,
b) Erzeugen eines Deskriptors, wobei der Deskriptor eine Folge von Wechselwirkungswerten ist, für jede dreidimensionale Konfiguration durch das Verfahren nach einem der Ansprüche 13 bis 18,
c) Verbinden aller Deskriptoren mit einer gemessenen biologischen Aktivität,
d) Definieren einer Vielzahl an Gleichungen, wobei jede Gleichung einer Molekülart der Reihe entspricht, wobei in jeder Gleichung die gemessene biologische Aktivität der entsprechenden Molekülart gleich einer gewichteten linearen Kombination der Wechselwirkungswerte eingesellt wird, die gewichtete lineare Kombination durch unbekannte Koeffizienten gewichtet wird und die Vielzahl an Gleichungen ein System aus Gleichungen bilden, und
e) Finden einer zumindest annähernden Lösung für das System an Gleichungen, wobei die Lösung die Gruppe an Koeffizienten ist, welche dem am nächsten kommen, jede Gleichung zu erfüllen.

**21.** Computerprogrammprodukt, welches einen Softwarecode zum Implementieren eines der Verfahren nach den Ansprüchen 1 bis 20 beim Ausführen auf einem Rechensystem aufweist.

**22.** Maschinenlesbarer Datenträger, welcher das Computerprogramm nach Anspruch 21 speichert.

## Revendications

**1.** Procédé assisté par ordinateur pour produire un descripteur d'un objet tridimensionnel, ledit objet tridimensionnel étant sélectionné à partir du groupe constitué par :

1) une surface obtenue à partir d'une poche de biomolécule,
2) une configuration tridimensionnelle d'une espèce moléculaire, et
3) une surface catalytique,

ledit objet tridimensionnel étant représenté par un ensemble de coordonnées, ledit descripteur étant une chaîne de valeurs d'interaction $V_i$, ledit procédé comprenant l'étape consistant à attribuer une ou plusieurs propriétés et une valeur $V_b$ pour chacune desdites une ou plusieurs propriétés au niveau de coordonnées choisies à l'intérieur dudit ensemble de coordonnées dudit objet tridimensionnel, **caractérisé en ce que** les étapes suivantes sont effectuées pour chaque cage d'un ensemble d'une ou plusieurs cages, ledit ensemble d'une ou plusieurs cages mettant en oeuvre un environnement artificiel, lesdites une ou plusieurs cages ayant chacune une forme tridimensionnelle sur la surface de laquelle un ensemble de points est positionné, les mêmes une ou plusieurs propriétés telles qu'attribuées audit objet tridimensionnel et une valeur $V_c$ pour chacune desdites une ou plusieurs propriétés étant attribuées à chacun desdits points :

i) enfermer totalement ledit objet tridimensionnel dans ladite cage,
ii) pour chaque propriété, tout en conservant ledit objet tridimensionnel totalement enfermé dans ladite cage en faisant varier une ou plusieurs dimensions de ladite cage, déterminer la valeur d'interaction la plus basse possible $V_i$ ou la valeur d'interaction la plus élevée $V_i$ ou une valeur d'interaction $V_i$ dont on a fait la moyenne sur toutes les orientations résultant de l'interaction entre lesdites coordonnées choisies dudit objet tridimensionnel et lesdits points de ladite cage en échantillonnant sur l'espace rotatif entier l'orientation relative entre l'objet tridimensionnel et ladite cage, et
iii) attribuer chacune des valeurs d'interaction les plus basses possibles obtenues $V_i$ ou des valeurs d'interaction les plus élevées $V_i$ ou de la valeur d'interaction $V_i$ dont on a fait la moyenne sur toutes les orientations à une position distincte dans ledit descripteur.

**2.** Procédé selon la revendication 1, **caractérisé en ce que** ladite valeur $V_c$ est l'une ou l'autre de +1, 0 ou -1.

**3.** Procédé selon la revendication 2, **caractérisé en ce que** ladite valeur $V_c$ est l'une ou l'autre de +1 ou -1.

**4.** Procédé selon l'une quelconque des revendications 1 à 3, **caractérisé en ce que** la somme des valeurs $V_c$ pour chaque cage est nulle.

**5.** Procédé selon l'une quelconque des revendications précédentes, **caractérisé en ce que** ladite valeur d'interaction $V_i$ est donnée par la formule suivante :

$$V_i = \sum_{h-i}^{\text{nCoordonnées}} \sum_{c=i}^{\text{nPo ints}} \frac{f(V_b)\, g(V_C)}{h(d_{bc})}$$

dans laquelle nCoordonnées est le nombre total desdites coordonnées choisies dans ledit objet tridimensionnel, nPoints est le nombre total desdits points, $V_b$ est la valeur de la propriété à la coordonnée de rang b, $V_c$ est la valeur de la propriété au point de cage de rang c, $d_{bc}$ est la distance entre la coordonnée b et le point de cage c, et f(), g () et h() sont des fonctions de transformation mathématiques.

6. Procédé selon l'une quelconque des revendications précédentes, **caractérisé en ce que** pour la minimisation de la valeur d'interaction $V_i$, les dimensions de la cage sont sélectionnées de sorte qu'au moins deux positions sur l'objet sont plus proches que 1 nm de la cage.

7. Procédé selon l'une quelconque des revendications précédentes, **caractérisé en ce que** ledit ensemble de points comprend entre quatre et douze points.

8. Procédé selon l'une quelconque des revendications précédentes, **caractérisé en ce qu'**au moins une desdites cages est stéréospécifique.

9. Procédé selon l'une quelconque des revendications précédentes, **caractérisé en ce que** lesdites une ou plusieurs cages sont sélectionnées à partir de cages cubiques sur la surface desquelles ledit ensemble de points est constitué par :

   a) quatre points occupant la moitié des angles de chaque face de ladite cage cubique, ou
   b) six points occupant le centre de chaque face de ladite cage cubique, ou
   c) huit points occupant tous les angles de ladite cage cubique, ou
   d) douze points occupant le milieu de chaque bord de ladite cage cubique.

10. Procédé selon l'une quelconque des revendications précédentes, **caractérisé en ce que** lesdites unes ou plusieurs cages sont au nombre de quatre ou plus.

11. Procédé selon l'une quelconque des revendications précédentes, **caractérisé en ce que** chaque valeur $V_b$ est normalisée avant d'effectuer l'étape ii).

12. Procédé selon l'une quelconque des revendications précédentes, **caractérisé en ce qu'**au moins une desdites une ou plusieurs cages est en forme de cube.

13. Procédé selon l'une quelconque des revendications précédentes, **caractérisé en ce que** ledit objet tridimensionnel est une configuration tridimensionnelle d'une espèce moléculaire, et dans lequel lesdites coordonnées choisies sont des positions atomiques choisies.

14. Procédé selon l'une quelconque des revendications précédentes, **caractérisé en ce que** lesdites positions atomiques choisies sont toutes les positions atomiques de ladite configuration tridimensionnelle de ladite espèce moléculaire.

15. Procédé selon la revendication 5, **caractérisé en ce que** ledit objet tridimensionnel est une configuration tridimensionnelle d'une espèce moléculaire, nCoordonnées est le nombre total desdites positions atomiques choisies dans ladite configuration tridimensionnelle d'une espèce moléculaire, $V_b$ est la valeur de la propriété à la position atomique de rang b, $d_{bc}$ est la distance entre l'atome b et le point de cage c.

16. Procédé selon l'une quelconque des revendications 13 à 15, **caractérisé en ce que** ladite configuration tridimensionnelle est déterminée soit par une simulation informatique, soit par un ou plusieurs moyens d'analyse en laboratoire ou par une combinaison d'une simulation informatique et d'un ou plusieurs moyens d'analyse en laboratoire.

17. Procédé selon l'une quelconque des revendications 13 à 16, comprenant en outre l'étape consistant à optimiser la conformation de ladite configuration tridimensionnelle de ladite espèce moléculaire après l'étape i) et avant l'étape iii), de façon à minimiser la valeur d'interaction calculée $V_i$ résultant de l'interaction entre ladite configuration tridimensionnelle de ladite espèce moléculaire et ladite cage.

18. Procédé selon l'une quelconque des revendications précédentes à utiliser dans le criblage virtuel de bases de données moléculaires, dans la synthèse virtuelle de molécules ayant des propriétés prédéterminées, et dans la compréhension et dans l'obtention de structure / relations de telles espèces moléculaires.

19. Procédé d'évaluation de la similitude entre un objet tridimensionnel de référence et un objet tridimensionnel de test en calculant la similitude entre les descripteurs correspondants produits par le procédé selon l'une quelconque des revendications précédentes.

**20.** Procédé de production d'une relation d'activité de structure quantitative tridimensionnelle, 3D-QSAR, d'une série d'espèces moléculaires comprenant les étapes consistant à :

a) obtenir une configuration tridimensionnelle pour chacune desdites espèces moléculaires,
b) produire un descripteur, ledit descripteur étant une chaîne de valeurs d'interaction, pour chaque configuration tridimensionnelle, par le procédé selon l'une quelconque des revendications 13 à 18,
c) associer chacun desdits descripteurs à une activité biologique mesurée,
d) définir une pluralité d'équations, chaque équation correspondant à une espèce moléculaire de la série, dans lesquelles dans chaque équation ladite activité biologique mesurée de l'espèce moléculaire correspondante est fixée égale à une combinaison linéaire pondérée desdites valeurs d'interaction, ladite combinaison linéaire pondérée étant pondérée par des coefficients inconnus, ladite pluralité d'équations formant un système d'équations, et
e) rechercher une solution au moins approximative au système d'équations, ladite solution étant l'ensemble de coefficients qui parviennent le plus à rendre chaque équation vraie.

**21.** Produit formant programme informatique comprenant un code logiciel pour mettre en oeuvre n'importe lequel des procédés selon les revendications 1 à 20 quand il est exécuté sur un système de calcul.

**22.** Support de données pouvant être exploitées par une machine stockant le programme informatique selon la revendication 21.

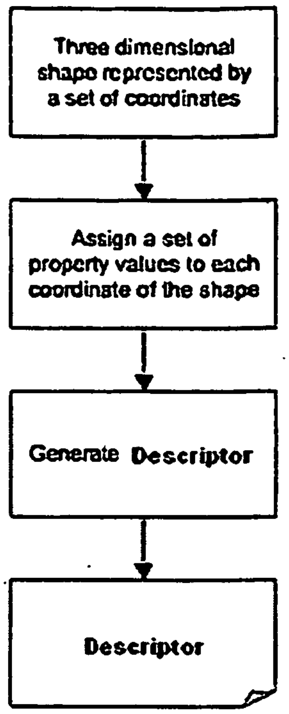
Three dimensional shape represented by a set of coordinates
Assign a set of property values to each coordinate of the shape
Generate Descriptor
Descriptor

FIGURE 1

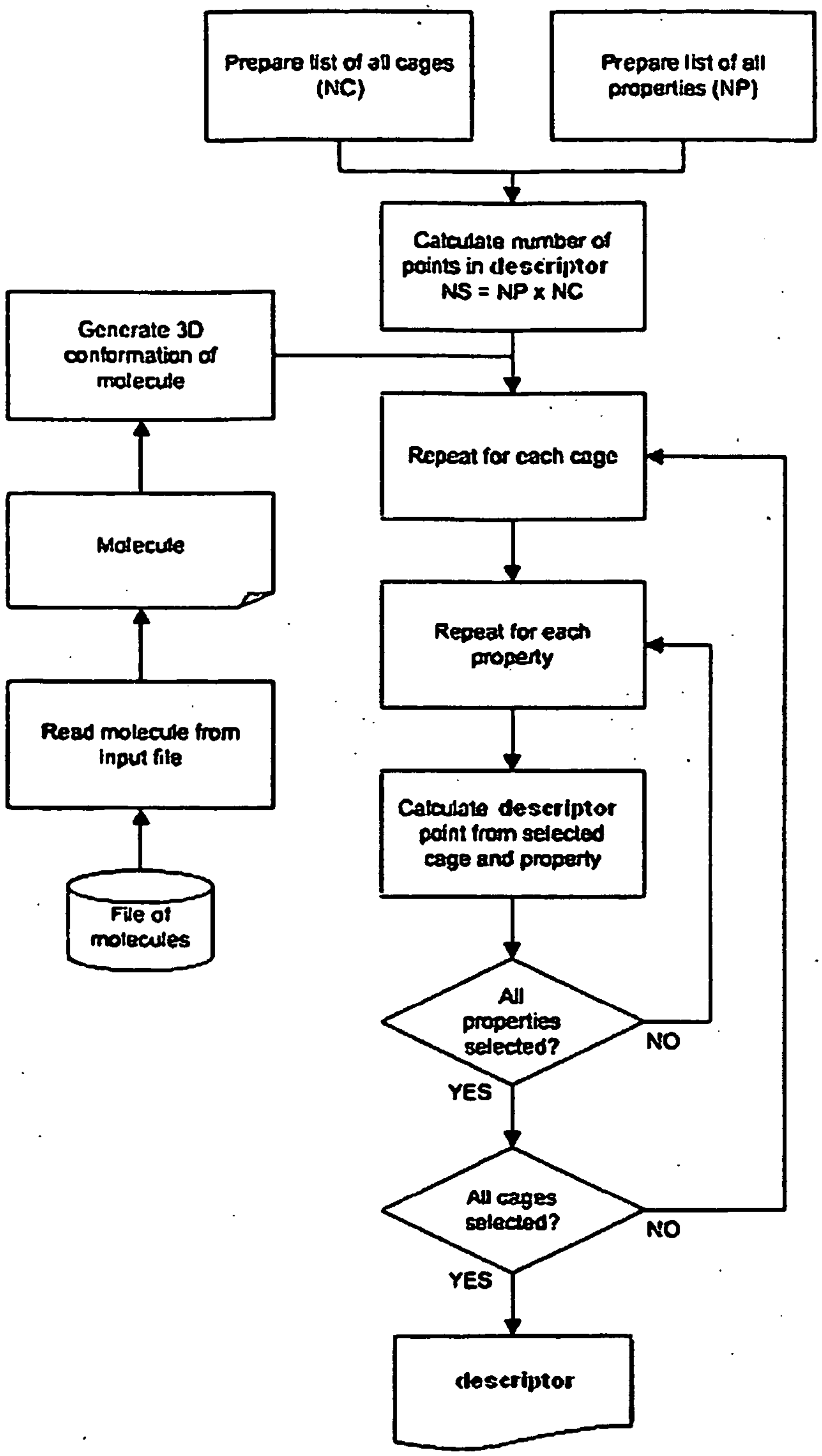
Prepare list of all cages (NC)
Prepare list of all properties (NP)
Calculate number of points in descriptor NS = NP x NC
Generate 3D conformation of molecule
Repeat for each cage
Molecule
Repeat for each property
Read molecule from input file
Calculate descriptor point from selected cage and property
File of molecules
All properties selected?
NO
YES
All cages selected?
NO
YES
descriptor

FIGURE 2

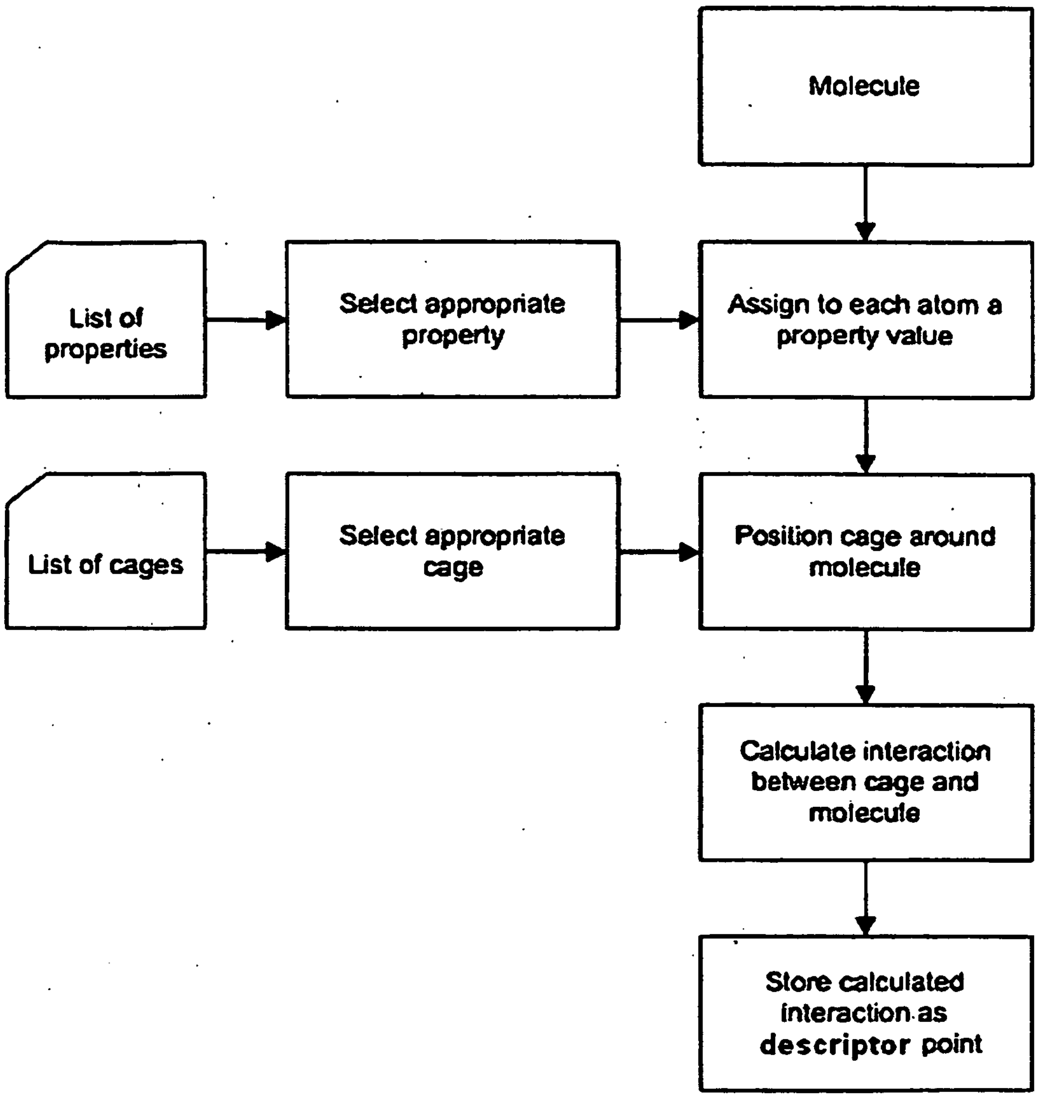
Molecule
List of properties
Select appropriate property
Assign to each atom a property value
List of cages
Select appropriate cage
Position cage around molecule
Calculate interaction between cage and molecule
Store calculated interaction as descriptor point

FIGURE 3

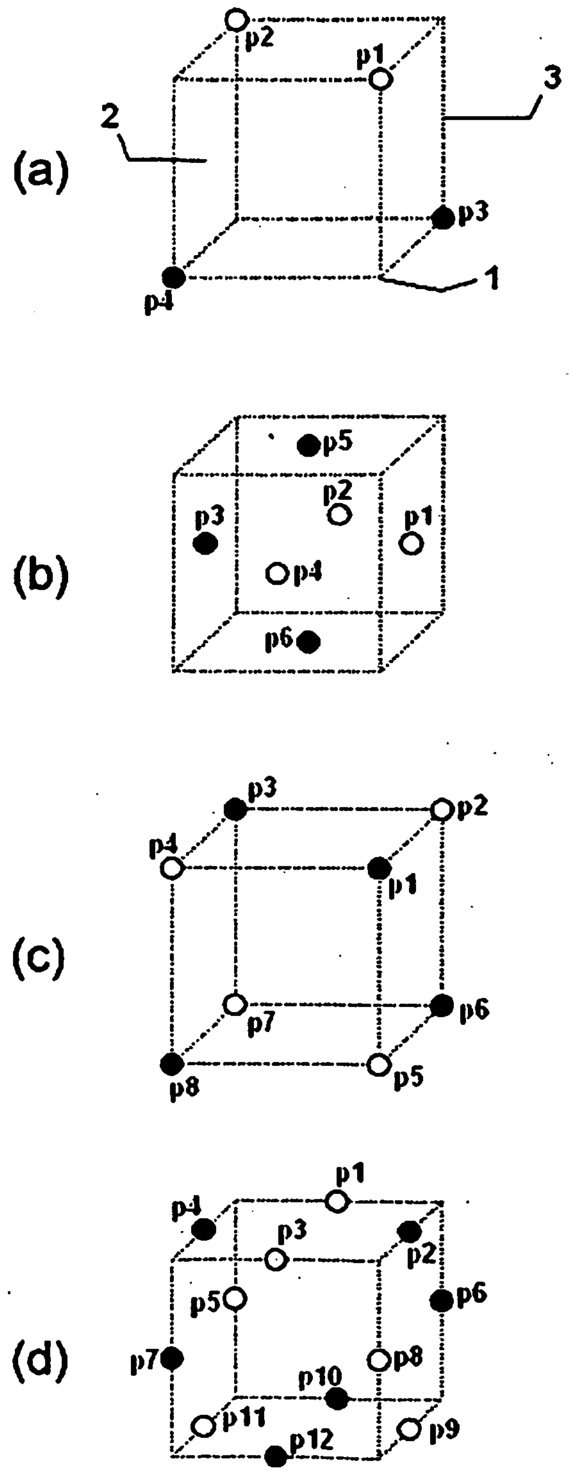
(a)
p1
p2
p3
p4
1
2
3
(b)
p1
p2
p3
p4
p5
p6
(c)
p1
p2
p3
p4
p5
p6
p7
p8
(d)
p1
p2
p3
p4
p5
p6
p7
p8
p9
p10
p11
p12

FIGURE 4

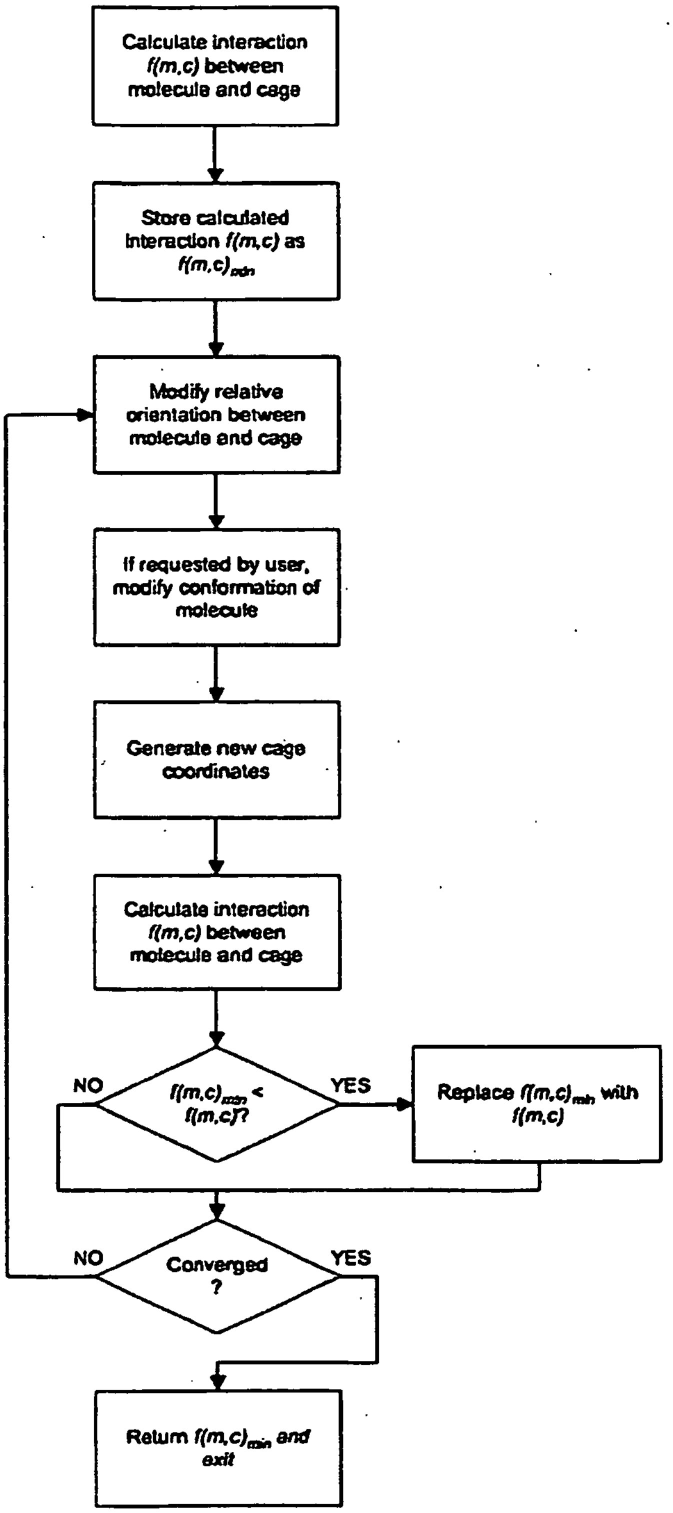
Calculate interaction f(m,c) between molecule and cage
Store calculated interaction f(m,c) as f(m,c)min
Modify relative orientation between molecule and cage
If requested by user, modify conformation of molecule
Generate new cage coordinates
Calculate interaction f(m,c) between molecule and cage
NO
f(m,c)min < f(m,c)?
YES
Replace f(m,c)min with f(m,c)
NO
Converged ?
YES
Return f(m,c)min and exit

FIGURE 5

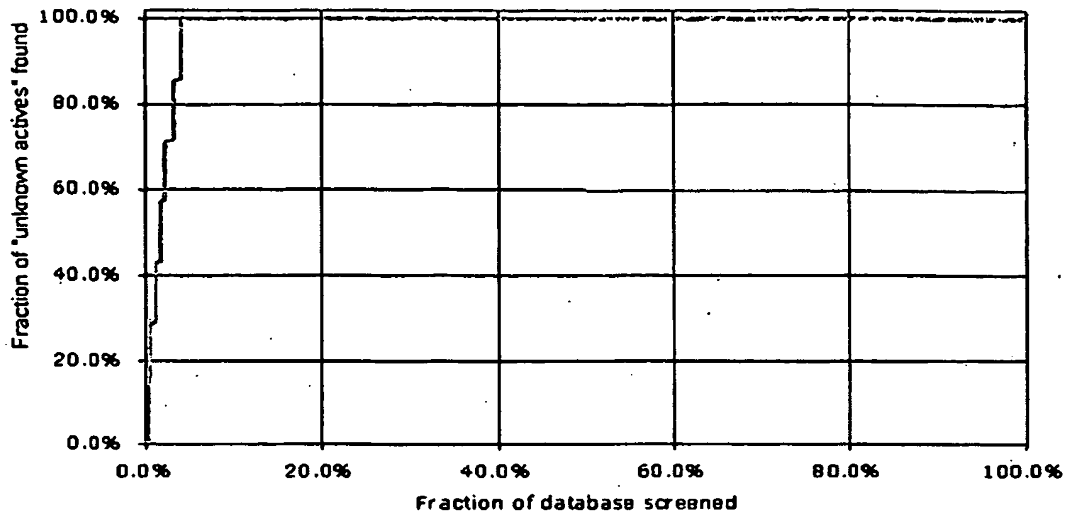


FIGURE 6

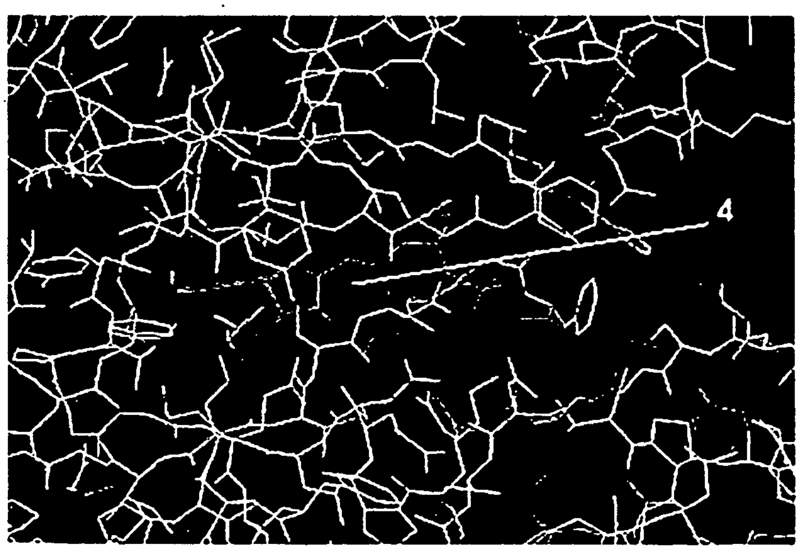


FIGURE 7(a)

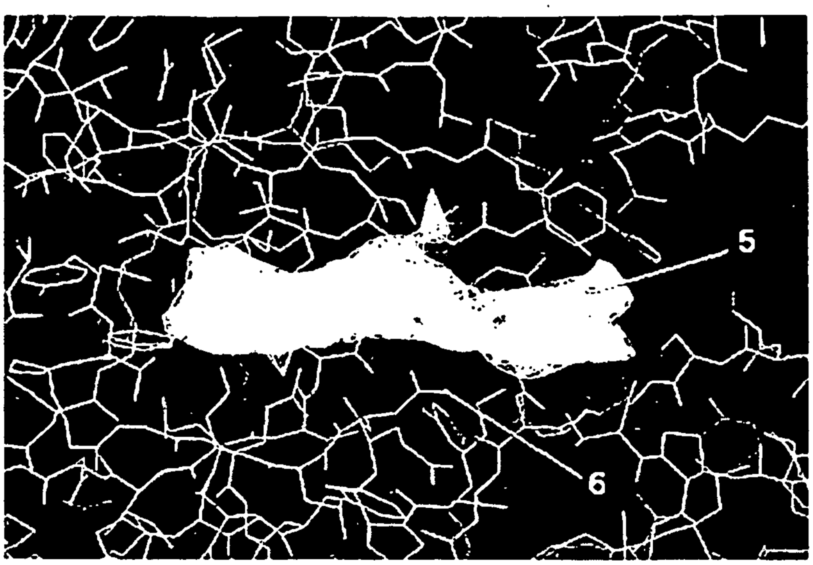


FIGURE 7(b)

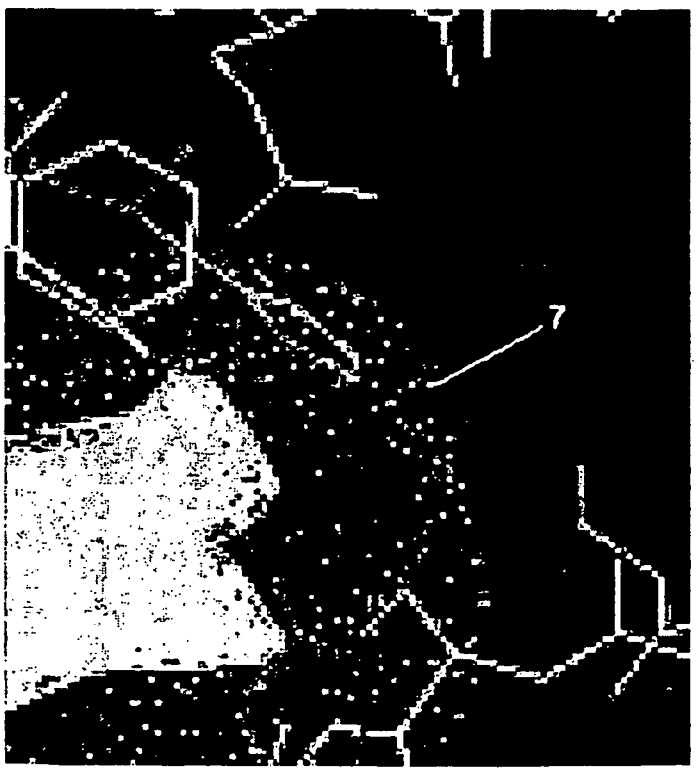


FIGURE 7(c)

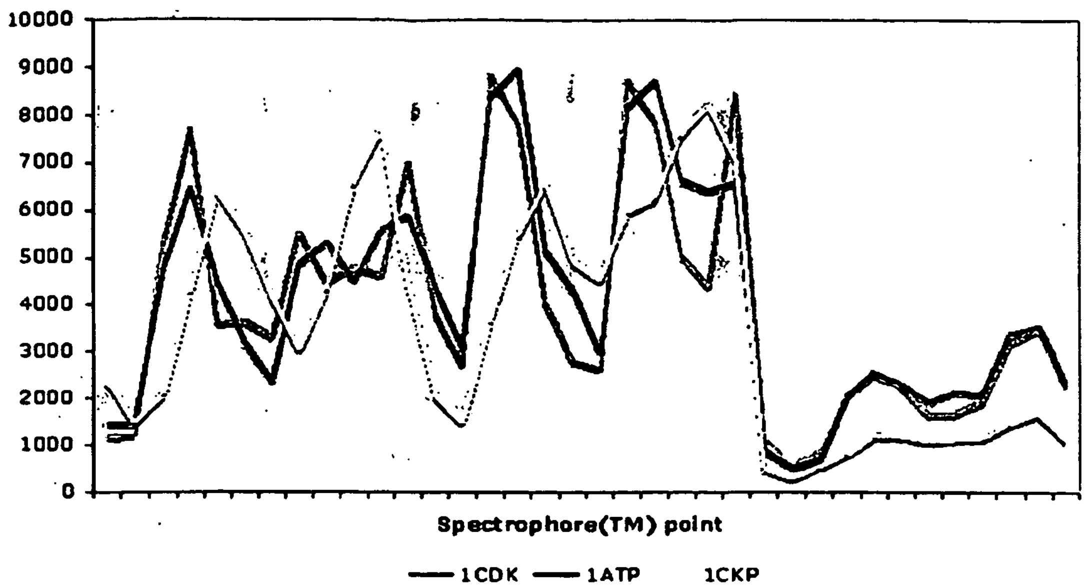
10000
9000
8000
7000
6000
5000
4000
3000
2000
1000
0
Spectrophore(TM) point
1CDK
1ATP
1CKP

FIGURE 8

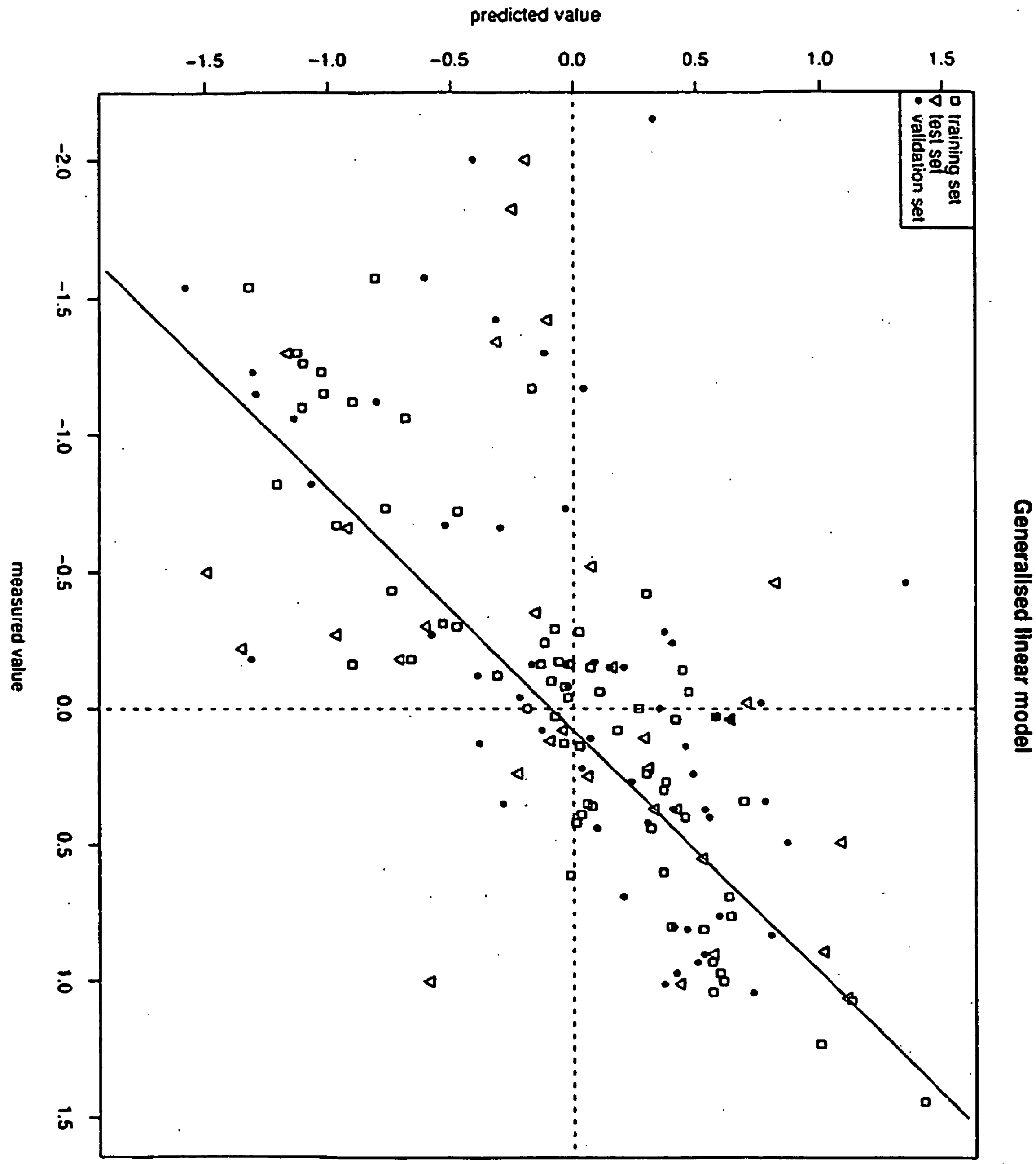
predicted value
measured value
Generalised linear model
training set
test set
validation set
-1.5
-1.0
-0.5
0.0
0.5
1.0
1.5
-2.0
-1.5
-1.0
-0.5
0.0
0.5
1.0
1.5

FIGURE 9(a)

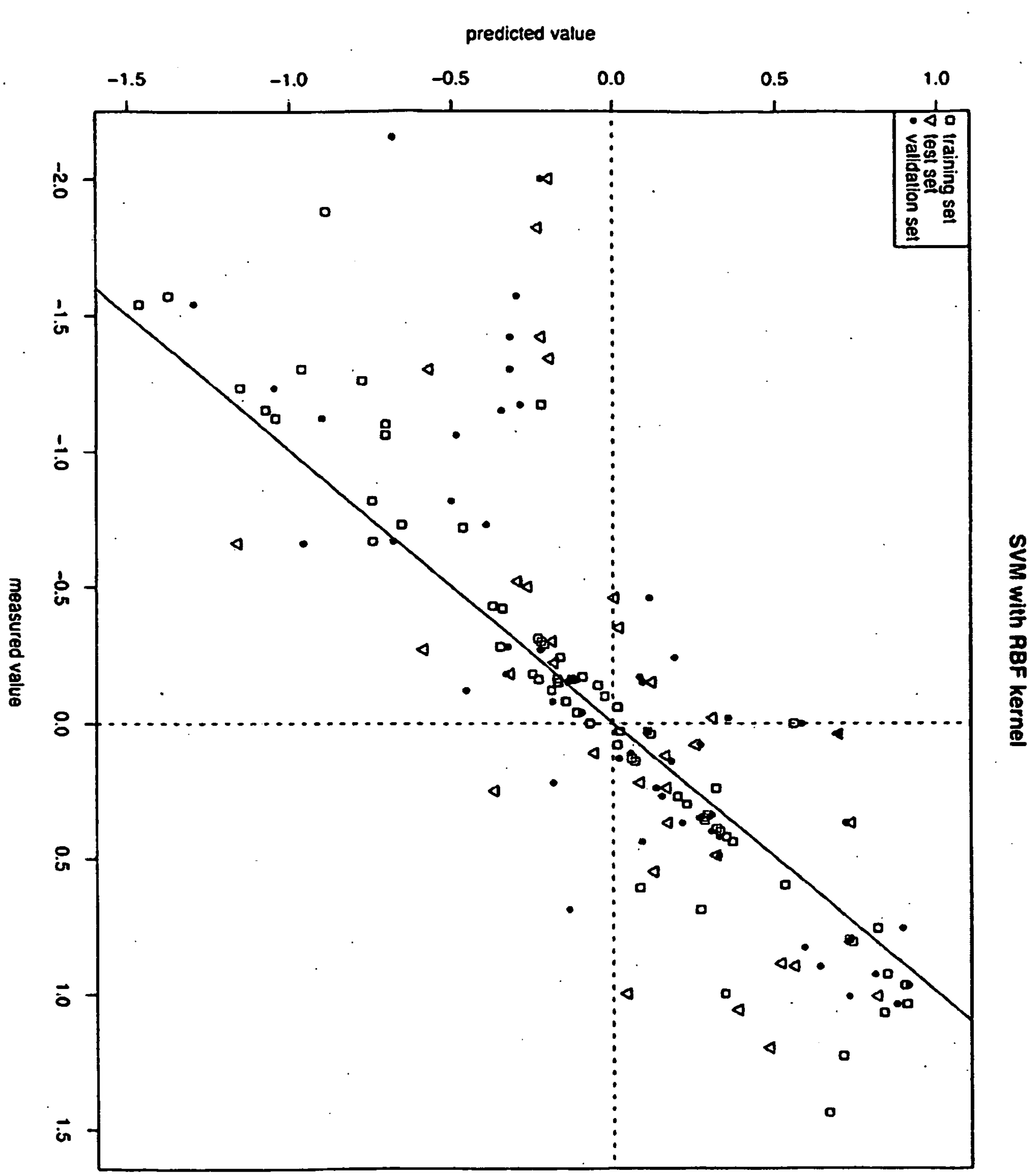
SVM with RBF kernel
predicted value
-1.5
-1.0
-0.5
0.0
0.5
1.0
measured value
-2.0
-1.5
-1.0
-0.5
0.0
0.5
1.0
1.5
training set
test set
validation set

FIIGURE 9(b)

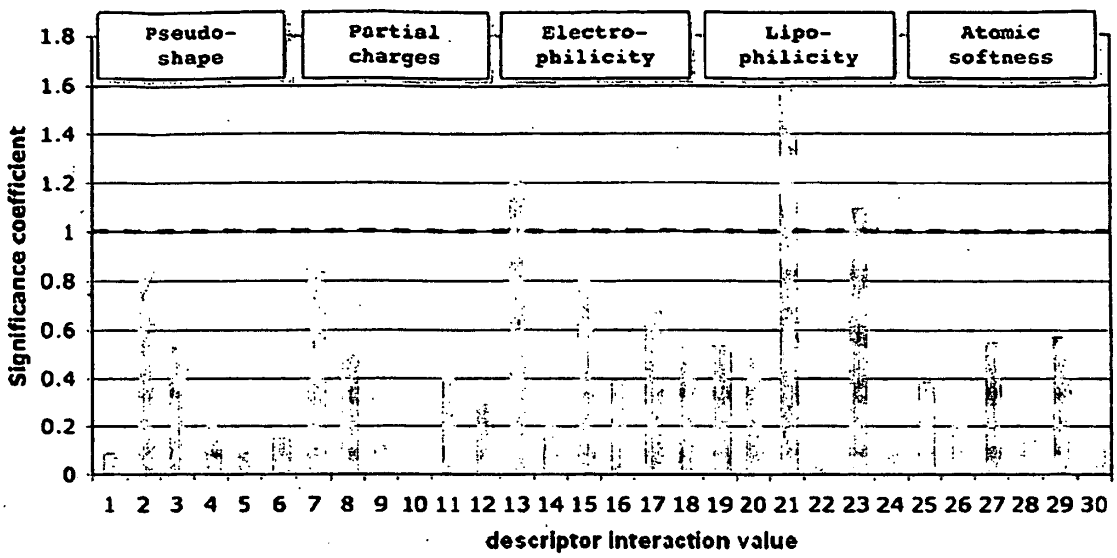
Pseudo-shape
Partial charges
Electro-philicity
Lipo-philicity
Atomic softness
1.8
1.6
1.4
1.2
1
0.8
0.6
0.4
0.2
0
Significance coefficient
1 2 3 4 5 6 7 8 9 10 11 12 13 14 15 16 17 18 19 20 21 22 23 24 25 26 27 28 29 30
descriptor interaction value

FIGURE 10

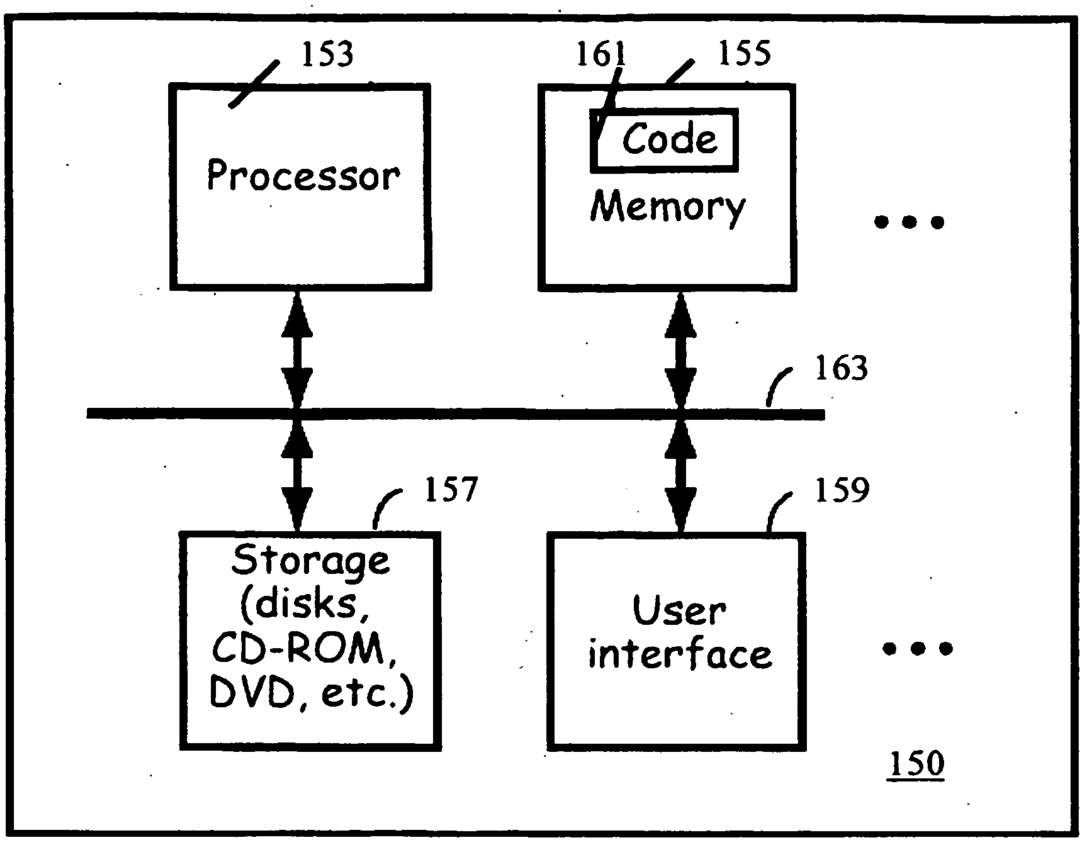
153
161
155
Processor
Code
Memory
163
157
159
Storage
(disks,
CD-ROM,
DVD, etc.)
User
interface
150

FIGURE 11

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 5025388 A
- WO 2004023349 A


### Non-patent literature cited in the description


- **SADOWSKI et al.** *Chem. Rev.,* 1993, vol. 7, 2567-2581
- **BOSTRÖM et al.** *J. Mol. Graph. Mod.,* 2003, vol. 21, 449-462
- *JOURNAL OF PHYSICAL CHEMISTRY A ACS USA,* 30 January 2003, vol. 107 (4), 542-553
- **BULTINCK et al.** *J. Phys. Chem. A.,* 2002, vol. 106, 7895-7901
- *J. Chem. Inf. Comput. Sci.,* 2003, vol. 43, 422-428
- **HEIDEN et al.** *J. Comput. Aided Mol. Des.,* 1993, vol. 7, 503-514
- **MANNHOLD et al.** *J. Pharm. Sci.,* 1995, vol. 84, 1410-1419
- **GAILLARD et al.** *J. Comput. Aided Mol. Des.,* 1994, vol. 8, 83-96
- **ERTL et al.** *J. Med. Chem.,* 2000, vol. 43, 3714-3717
- **REY et al.** *J. Mol. Graph. Model.,* 2001, vol. 19, 521-535
- **KIER ; HALL.** *Pharm. Res.,* 1990, vol. 7, 801-807
- **HALL ; KIER.** *J. Chem. Inf. Comput. Sci.,* 1995, vol. 35, 1039-1045
- **VINTER et al.** *J. Comput.-Aided Mol. Des.,* 1995, vol. 9, 297-307
- **MESTRES et al.** *J. Mol. Graph. Model.,* 1997, vol. 15, 114-121
- **LASKOWSKI.** *J. Mol. Graph.,* 1995, vol. 13, 323-330
- **RICHARDS.** *Ann. Rev. Biophys. Bioeng.,* 1977, vol. 6, 151-176
- **SANDBERG et al.** *J. Med. Chem.,* 1998, vol. 41, 2481-2491
- **BAYLY et al.** *J. Phys. Chem.,* 1993, vol. 97, 10269-10280
- **MCELROY.** *J. Med. Chem.,* 2003, vol. 46, 1066-1080
- **ROSE.** *J. Chem. Inf. Comput. Sci.,* 2002, vol. 42, 651-666